# EUROPEAN PATENT APPLICATION

(11) **EP 1 262 196 A2**
(43) Date of publication of application: **04.12.2002**
(21) Application number: 02253105.7
(22) Date of filing: 02.05.2002
(51) Int. Cl.: A61K 45/06, A61P 25/32

(54) **Combination of a monoamine reuptake inhibitor and an opioid antagonist for use in alcoholism and alcohol dependence**

(30) Priority: 23.05.2001 US 293088 P
(71) Applicant: Pfizer Products Inc., Groton, Connecticut 06340 (US)
(72) Inventor: Howard, Jr., Harry Ralph, Groton, Connecticut 06340 (US)
(74) Representative: Hayles, James Richard

(57) **Abstract**

The present invention relates to a method of treating alcoholism or alcohol dependence in a mammal, including a human, by administering to the mammal a monoamine reuptake inhibitor in combination with an opioid antagonist. It also relates to pharmaceutical compositions containing a pharmaceutically acceptable carrier, a monoamine reuptake inhibitor and an opioid antagonist.

## Description

### Background Of The Invention

The present invention relates to a method of treating alcoholism or alcohol dependence in a mammal, including a human, by administering to the mammal a biaryl ether derivative, as described below, in combination with an opioid antagonist. This invention also relates to pharmaceutical compositions containing a pharmaceutically acceptable carrier, a biaryl ether derivative, as described below, and an opioid antagonist.

The biaryl ether derivatives referred to above that are employed in the methods and pharmaceutical compositions of this invention exhibit activity as monoamine (*e.g.*, serotonin, dopamine, norepinephrine) reuptake inhibitors. These biaryl ether derivatives are referred to in United States Patent Application No. 09/692,335, filed October 19, 2000, and in International Patent Application No. WO 00/50380, published August 31, 2000.

### Summary Of The Invention

The present invention relates to a pharmaceutical composition for the treatment of alcoholism or alcohol dependence in a mammal, including a human, comprising: (a) an opioid antagonist, or pharmaceutically acceptable salt thereof; (b) a compound of the formula I, as depicted and defined below, 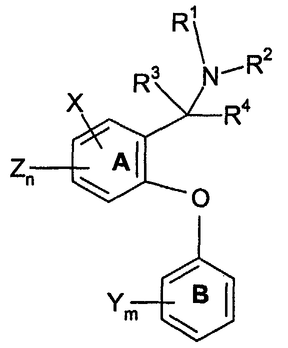 or pharmaceutically acceptable salt thereof, wherein phenyl ring A and phenyl ring B can each, independently, be replaced by a naphthyl group, and wherein when phenyl ring A is replaced by a naphthyl group, the ethereal oxygen of structure I and the carbon to which R³, R⁴ and NR¹R² are attached, are attached to adjacent ring carbon atoms of the naphthyl group and neither of said adjacent ring carbon atoms is also adjacent to a fused ring carbon atom of said naphthyl group;
n and m are selected, independently, from one, two and three;
R¹ and R² are selected, independently, from hydrogen, (C₁-C₄)alkyl, (C₂-C₄)alkenyl, and (C₂-C₄)alkynyl, or R¹ and R², together with the nitrogen to which they are attached, form a four to eight membered saturated ring containing one or two heteroatoms, including the nitrogen to which R¹ and R² are attached, wherein the second heteroatom, when present, is selected from oxygen, nitrogen and sulfur, and wherein said ring may optionally be substituted at available binding sites with from one to three substituents selected, independently, from hydroxy and (C₁-C₆)alkyl;
R³ and R⁴ are selected, independently, from hydrogen and (C₁-C₄) alkyl optionally substituted with from one to three fluorine atoms, or R³ and R⁴ together with the carbon to which they are attached form a four to eight membered saturated carbocyclic ring, and wherein said ring may optionally be substituted at available binding sites with from one to three substituents selected, independently, from hydroxy and (C₁-C₆)alkyl;
or R² and R³, together with the nitrogen to which R² is attached and the carbon to which R³ is attached, form a four to eight membered saturated ring containing one or two heteroatoms, including the nitrogen to which R² is attached, wherein the second heteroatom, when present, is selected from oxygen, nitrogen and sulfur, and wherein said ring may optionally be substituted at available binding sites with from one to three substituents selected, independently, from hydroxy and (C₁-C₆)alkyl;
each X is selected, independently, from phenyl, heteroaryl (*e.g.*, furan, thiophene, pyrrole, thiazole, isothiazole, oxazole, isoxazole, imidazole, 1,2,4-oxadiazole, 1,2,4-thiadiazole, 1,2,4-triazole, 1,2,3,-triazole, tetrazole, pyridine, pyrimidine, pyrazine, quinoline, isoquinoline, quinazoline, quinoxaline, benzothiophene, benzofuran, benzimidazole, benzisoxazole, benzisothiazole and indole) and heterocyclic (e.g., imidazolidine, oxazolidine, thiazolidine, pyrrolidine, piperidine, morpholine) groups, as defined below, and may be further substituted by hydrogen, halo (*i.e*., fluorine, chlorine, bromine, iodine), (C₁-C₄)alkyl optionally substituted with from one to three fluorine atoms, (C₁-C₄)alkoxy optionally substituted with from one to three fluorine atoms, cyano, nitro, amino, hydroxy, carbonyl, (C₁-C₄)alkylamino, di[(C₁-C₄)alkyl]amino, NR⁵(C=O)(C₁-C₄)alkyl, SO₂NR⁵R⁶ or SOₚ(C₁-C₆)alkyl, wherein R⁵ and R⁶ are selected, independently, from hydrogen and (C₁-C₆)alkyl, and p is zero, one or two;
each Y is selected, independently, from hydrogen, halo (*i.e*., chloro, fluoro, bromo or iodo), (C₁-C₄)alkyl optionally substituted with from one to three fluorine atoms, (C₁-C₄)alkoxy optionally substituted with from one to three fluorine atoms, cyano, nitro, amino, (C₁-C₄)alkylamino, di-[(C₁-C₄)alkyl]amino, NR⁵(C=O)(C₁-C₄)alkyl, SO₂NR⁵R⁶ and SOₚ(C₁-C₆)alkyl, wherein R⁵ and R⁶ are selected, independently, from hydrogen and (C₁-C₆)alkyl, and p is zero, one or two; and
each Z is selected independently from hydrogen, halo (*i.e*., chloro, fluoro, bromo or iodo), (C₁-C₄)alkyl optionally substituted with from one'to three fluorine atoms, and (C₁-C₄)alkoxy; and (c) a pharmaceutically acceptable carrier; wherein the active agents "a" and "b" above are present in amounts that render the composition effective in treating alcoholism or alcohol dependence.

This invention also relates to a method of treating alcoholism or alcohol dependence in a mammal, including a human, comprising administering to said mammal a pharmaceutical composition comprising: (a) an opioid antagonist, or pharmaceutically acceptable salt thereof; (b) a compound of the formula I, as defined above, or pharmaceutically acceptable salt thereof; and (c) a pharmaceutically acceptable carrier; wherein the active agents "a" and "b" above are present in amounts that render the composition effective in treating alcoholism or alcohol dependence.

This invention also relates to a method of treating alcoholism or alcohol dependence in a mammal, including a human, comprising administering to said mammal: (a) an opioid antagonist, or pharmaceutically acceptable salt thereof; and (b) a compound of the formula I, as defined above, or pharmaceutically acceptable salt thereof; wherein the active agents "a" and "b" above are present in amounts that render the combination of the two agents effective in treating alcoholism or alcohol dependence.

The present invention also relates to a pharmaceutical composition for the treatment of alcoholism or alcohol dependence in a mammal, including a human, comprising: (a) an opioid antagonist, or pharmaceutically acceptable salt thereof; (b) a compound of the formula XXX, as depicted and defined below, 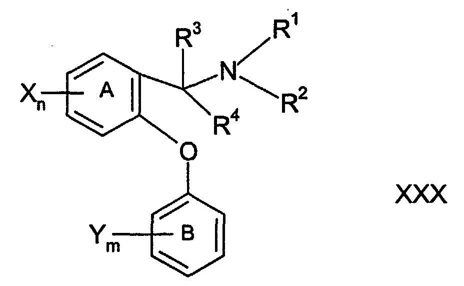 or pharmaceutically acceptable salt thereof, wherein phenyl ring A and phenyl ring B can each, independently, be replaced by a naphthyl group, and wherein when phenyl ring A is replaced by a naphthyl group, the ethereal oxygen of structure I and the carbon to which R³, R⁴ and NR¹R² are attached, are attached to adjacent ring carbon atoms of the naphthyl group and neither of said adjacent ring carbon atoms is also adjacent to a fused ring carbon atom of said naphthyl group;
n and m are, selected, independently, from one, two and three;
R¹ and R² are selected, independently, from hydrogen, (C₁-C₄)alkyl, (C₂-C₄)alkenyl, and (C₂-C₄)alkynyl, or R¹ and R², together with the nitrogen to which they are attached, form a four to eight membered saturated ring containing one or two heteroatoms, including the, nitrogen to which R¹ and R² are attached, wherein the second heteroatom, when present, is selected from oxygen, nitrogen and sulfur, with the proviso that said ring can not contain two adjacent oxygen atoms or two adjacent sulfur atoms, and wherein said ring may optionally be substituted at available binding sites with from one to three substituents selected, independently, from hydroxy and (C₁-C₆)alkyl;
R³ and R⁴ are selected, independently, from hydrogen and (C₁-C₄) alkyl optionally substituted with from one to three fluorine atoms, or R³ and R⁴, together with the carbon to which they are attached, form a four to eight membered saturated carbocyclic ring, and wherein said ring may optionally be substituted at available binding sites with from one to three substituents selected, independently, from hydroxy and (C₁-C₆)alkyl;
or R² and R³, together with the nitrogen to which R² is attached and the carbon to which R³ is attached, form a four to eight membered saturated ring containing one or two heteroatoms, including the nitrogen to which R² is attached, wherein the second heteroatom, when present, is selected from oxygen, nitrogen and sulfur, with the proviso that said ring can not contain two adjacent oxygen atoms or two adjacent sulfur atoms, and wherein said ring may optionally be substituted at available binding sites with from one to three substituents selected, independently, from hydroxy and (C₁-C₆)alkyl;
each X is selected, independently, from hydrogen, halo (*i.e*., chloro, fluoro, bromo or iodo), (C₁-C₄)alkyl optionally substituted with from one to three fluorine atoms, (C₁-C₄)alkoxy optionally substituted with from one to three fluorine atoms, cyano, nitro, amino, (C₁-C₄)alkylamino, di-[(C₁-C₄)alkyl]amino, NR⁵(C=O)(C₁-C₄)alkyl, SO₂NR⁵R⁶ and SOₚ(C₁-C₆)alkyl, wherein R⁵ and R⁶ are selected, independently, from hydrogen and (C₁-C₆)alkyl, and p is zero, one or two; and
each Y is selected, independently, from hydrogen, (C₁-C₆)alkyl and halo;
with the proviso that: (1) no more than one of NR¹R², CR³R⁴ and R²NCR³ can form a ring; and (2) at least one X must be other than hydrogen when (i) R³ and R⁴ are both hydrogen, (ii) R¹ and R² are selected, independently, from hydrogen and (C₁-C₄)alkyl, and (iii) ring B is mono- or disubstituted with, respectively, one or two halo groups; and (c) a pharmaceutically acceptable carrier; wherein the active agents "a" and "b" above are present in amounts that render the composition effective in treating alcoholism or alcohol dependence.

This invention also relates to a method of treating alcoholism or alcohol dependence in a mammal, including a human, comprising administering to said mammal a pharmaceutical composition comprising: (a) an opioid antagonist, or pharmaceutically acceptable salt thereof; (b) a compound of the formula XXX, as defined above, or pharmaceutically acceptable salt thereof; and (c) a pharmaceutically acceptable carrier; wherein the active agents "a" and "b" above are present in amounts that render the composition effective in treating alcoholism or alcohol dependence.

This invention also relates to a method of treating alcoholism or alcohol dependence in a mammal, including a human, comprising administering to said mammal: (a) an opioid antagonist, or pharmaceutically acceptable salt thereof; and (b) a compound of the formula XXX, as defined above, or pharmaceutically acceptable salt thereof; wherein the active agents "a" and "b" above are present in amounts that render the combination of the two agents effective in treating alcoholism or alcohol dependence.

The present invention also relates to a pharmaceutical composition for the treatment of alcoholism or alcohol dependence in a mammal, including a human, comprising: (a) an opioid antagonist, or pharmaceutically acceptable salt thereof; (b) a compound of the formula XXXI or XXXII, respectively, as depicted below, 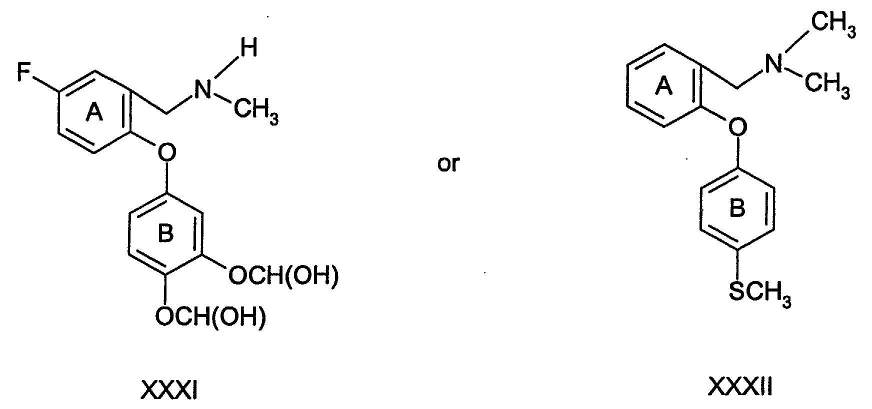 or pharmaceutically acceptable salt thereof; and (c) a pharmaceutically acceptable carrier; wherein the active agents "a" and "b" above are present in amounts that render the composition effective in treating alcoholism or alcohol dependence.

This invention also relates to a method of treating alcoholism or alcohol dependence in a mammal, including a human, comprising administering to said mammal a pharmaceutical composition comprising: (a) an opioid antagonist, or pharmaceutically acceptable salt thereof; (b) a compound of the formula XXXI or XXXII, respectively, as defined above, or pharmaceutically acceptable salt thereof; and (c) a pharmaceutically acceptable carrier; wherein the active agents "a" and "b" above are present in amounts that render the composition effective in treating alcoholism or alcohol dependence.

This invention also relates to a method of treating alcoholism or alcohol dependence in a mammal, including a human, comprising administering to said mammal: (a) an opioid antagonist, or pharmaceutically acceptable salt thereof; and (b) a compound of the formula XXXI or XXXII, respectively, as defined above, or pharmaceutically acceptable salt thereof; wherein the active agents "a" and "b" above are present in amounts that render the combination of the two agents effective in treating alcoholism or alcohol dependence.

It will be appreciated that when using a combination method of the present invention, referred to immediately above, both the monoamine reuptake inhibitor (*i.e*., the compound of formula I, XXX, XXXI or XXXII) and the opioid antagonist will be administered to a patient within a reasonable period of time. The compounds may be in the same pharmaceutically acceptable carrier and therefore administered simultaneously. Alternatively, they may be in separate pharmaceutical carriers such as conventional oral dosage forms that are administered simultaneously or within a time period that one of skill in the art would consider reasonable for effecting the desired combination therapy. By way of example, the opioid antagonist may be administered as a tablet and then, within a reasonable period of time, the monoamine reuptake inhibitor (*i.e.*, the compound of formula I, XXX, XXXI or XXXII) may be administered either as a tablet or a fast-dissolving oral dosage form.

The compositions of the present invention that contain a monoamine reuptake inhibitor (*i.e*., a compound of formula I, XXX, XXXI or XXXII) and an opioid antagonist are useful for the treatment of alcoholism or alcohol dependence. As used herein, the terms "alcoholism" and "alcohol dependence" include all the specific disorders, including types and subtypes, listed in the DSM-IV™ under the category of alcohol-related disorders (and more generally under the category of substance-related disorders). These include alcohol dependence, including the type with physiological dependence or without physiological dependence, and also including the type with early full remission, early partial remission, sustained full remission, sustained partial remission, on agonist therapy or in a controlled environment, alcohol abuse, alcohol intoxication and alcohol withdrawal, including the type with perceptual disturbances. As used herein, these terms also include alcohol-induced disorders such as alcohol intoxication delirium, alcohol withdrawal delirium, alcohol-induced persisting dementia, and alcohol-induced persisting amnestic disorder. In addition, as used herein, these terms also include alcohol-induced psychotic disorder with delusions, alcohol-induced psychotic disorder with hallucinations, alcohol-induced mood disorder, alcohol-induced anxiety disorder, alcohol-induced sexual dysfunction and alcohol-induced sleep disorder, all of which may be of the type with onset during intoxication or with onset during withdrawal. Alcohol-related disorder not otherwise specified is also included within the meaning of the above terms, as used herein. The meanings attributed to the different types and subtypes of alcohol-related disorders are as stated in the DSM-IV™. (See Diagnostic and Statistical Manual of Mental Disorders, Fourth Edition, (DSM-IV™), American Psychiatric Association, 1994, pp. 194-204 (and more generally, pp. 175-204)).

By the use of a combination of a monoamine reuptake inhibitor (*i.e*., a compound of formula I, XXX, XXXI or XXXII) and an opioid antagonist in accordance with the present invention, it is possible to treat alcoholism and/or alcohol dependence in patients for whom conventional therapy might not be wholly successful.

Unless otherwise indicated, the term "halo", as used in the compounds of formula I and XXX, respectively, includes fluoro, chloro, bromo and iodo.

Unless otherwise indicated, the term "alkyl", as used in the compounds of formula I and XXX, respectively, may be straight, branched or cyclic, and may include straight and cyclic moieties as well as branched and cyclic moieties.

Unless otherwise indicated, the term "alkenyl", as used in the compounds of formula I and XXX, respectively, includes unsaturated hydrocarbon radicals having one or more double bonds connecting two carbon atoms, wherein said hydrocarbon radical may have straight, branched or cyclic moieties or combinations thereof. Examples of "alkenyl" groups include, but are not limited to, ethenyl, propenyl, butenyl, pentenyl and dimethylpentyl, and include E and Z forms where applicable.

Unless otherwise indicated, the term "alkynyl", as used in the compounds of formula I and XXX, respectively, includes unsaturated hydrocarbon radicals having one or more triple bonds connecting two carbon atoms, wherein said hydrocarbon radical may have straight, branched or cyclic moieties or combinations thereof.

Unless otherwise indicated, the term "heteroaryl", as used in the compounds of formula I, refers to aromatic groups containing one or more heteroatoms (O, S, or N), preferably from one to four heteroatoms. Unless otherwise indicated, a multicyclic group containing one or more heteroatoms wherein at least one ring of the group is aromatic is also a "heteroaryl" group for purposes of the present invention. The heteroaryl groups of the compounds of this invention can also include ring systems substituted with one or more oxo moieties. Examples of heteroaryl groups are pyridinyl, pyridazinyl, imidazolyl, pyrimidinyl, pyrazolyl, triazolyl, pyrazinyl, quinolyl, isoquinolyl, tetrazolyl, furyl, thiophenyl, isoxazolyl, thiazolyl, oxazolyl, isothiazolyl, pyrrolyl, indolyl, benzimidazolyl, benzofuranyl, cinnolinyl, indazolyl, indolizinyl, phthalazinyl, triazinyl, isoindolyl, purinyl, oxadiazolyl, thiadiazolyl, furazanyl, benzofurazanyl, benzothiophenyl, benzotriazolyl, benzothiazolyl, benzisothiazolyl, benzoxazolyl, benzisoxazolyl, benzimidazolyl, quinazolinyl, quinoxalinyl, naphthyridinyl, dihydroquinolyl, tetrahydroquinolyl, dihydroisoquinolyl, tetrahydroisoquinolyl, benzofuryl, furopyridinyl, pyrolopyrimidinyl and azaindolyl.

Unless otherwise indicated, the term "heterocyclic", as used in the compounds of formula I, refers to non-aromatic cyclic groups containing one or more heteroatoms, preferably from one to four heteroatoms, each selected from O, S and N. Unless otherwise indicated, "heterocyclic" includes heterobicyclic groups. "Heterobicyclic" refers to non-aromatic two-ringed cyclic groups, wherein said rings share one or two atoms, and wherein at least one of the rings contains a heteroatom (O, S, or N). Unless otherwise indicated, for purposes of the present invention, heterobicyclic groups include spiro groups and fused ring groups. In one embodiment, each ring in the heterobicyclic group contains up to four heteroatoms (*i.e*., from zero to four heteroatoms, provided that at least one ring contains at least one heteroatom). The heterocyclic groups of this invention can also include ring systems substituted with one or more oxo moieties. Examples of heterocyclic groups include, but are not limited to, aziridinyl, azetidinyl, pyrrolidinyl, piperidinyl, azepinyl, piperazinyl, 1,2,3,6-tetrahydropyridinyl, oxiranyl, oxetanyl, tetrahydrofuranyl, tetrahydrothienyl, tetrahydropyranyl, tetrahydrothiopyranyl, oxazolidinyl, morpholino, thiomorpholino, thiazolidinyl, thioxanyl, pyrrolinyl, indolinyl, 2H-pyranyl, 4H-pyranyl, dioxanyl, 1,3-dioxolanyl, pyrazolinyl, dihydropyranyl, dihydrothienyl, dihydrofuranyl, pyrazolidinyl, imidazolinyl, imidazolidinyl, 3-azabicyclo[3.1.0]hexanyl, 3-azabicyclo[4.1.0]heptanyl, quinolizinyl, quinuclidinyl, 1,4-dioxasplro[4.5]decyl, 1,4-dioxaspiro[4.4]nonyl, 1,4-dioxaspiro[4.3]octyl and 1,4-dioxaspiro[4.2]heptyl.

The foregoing groups, heteroaryl or heterocyclic, may be C-attached or N-attached where such is possible. For instance, a group derived from pyrrole may be pyrrol-1-yl (N-attached) or pyrrol-3-yl (C-attached). The terms referring to the groups also encompass all possible tautomers.

When reference is made to SOₚ(C₁-C₆)alkyl, and p is two, this indicates a sulfone, in other words, S(=O)₂(C₁-C₆)alkyl.

In certain embodiments, this invention relates to the above pharmaceutical compositions for the treatment of alcoholism or alcohol dependence, and the above methods of treating alcoholism or alcohol dependence, wherein the compound of formula I, or pharmaceutically acceptable salt thereof, is a compound wherein ring B is phenyl, not replaced with a naphthyl group.

In other embodiments, this invention relates to the above pharmaceutical compositions for the treatment of alcoholism or alcohol dependence, and the above methods of treating alcoholism or alcohol dependence, wherein the compound of formula I, or pharmaceutically acceptable salt thereof, is a compound wherein phenyl ring B is replaced with a naphthyl group.

In preferred embodiments, this invention relates to the above pharmaceutical compositions for the treatment of alcoholism or alcohol dependence, and the above methods of treating alcoholism or alcohol dependence, wherein the compound of formula I, or pharmaceutically acceptable salt thereof, is a compound wherein, when ring B is phenyl, each Y is hydrogen or halo.

In more preferred embodiments, this invention relates to the above pharmaceutical compositions for the treatment of alcoholism or alcohol dependence, and the above methods of treating alcoholism or alcohol dependence, wherein the compound of formula I, or pharmaceutically acceptable salt thereof, is a compound wherein m is 1 or 2, and each Y is chlorine.

In other embodiments, this invention relates to the above pharmaceutical compositions for the treatment of alcoholism or alcohol dependence, and the above methods of treating alcoholism or alcohol dependence, wherein the compound of formula I, or pharmaceutically acceptable salt thereof, is as described above, but wherein X is selected from furan, thiophene, pyrrole, and 1,2,3-triazole, and wherein X may be further substituted as recited above.

In other embodiments, this invention relates to the above pharmaceutical compositions for the treatment of alcoholism or alcohol dependence, and the above methods of treating alcoholism or alcohol dependence, wherein the compound of formula I, or pharmaceutically acceptable salt thereof, is a compound wherein X is a lactam, for example 1-pyrrolidin-2-one or 1-piperidin-2-one, optionally substituted as recited above and attached to ring A through the lactam nitrogen.

In other embodiments, this invention relates to the above pharmaceutical compositions for the treatment of alcoholism or alcohol dependence, and the above methods of treating alcoholism or alcohol dependence, wherein the compound of formula I, or pharmaceutically acceptable salt thereof, is a compound wherein X is a tetrazole optionally substituted as recited above and attached to ring A through the tetrazole carbon.

In other embodiments, this invention relates to the above pharmaceutical compositions for the treatment of alcoholism or alcohol dependence, and the above methods of treating alcoholism or alcohol dependence, wherein the compound of formula I, or pharmaceutically acceptable salt thereof, is a compound as described above, but wherein each Z is selected from hydrogen and halo.

In other preferred embodiments, this invention relates to the above pharmaceutical compositions for the treatment of alcoholism or alcohol dependence, and the above methods of treating alcoholism or alcohol dependence, wherein the compound of formula I, or pharmaceutically acceptable salt thereof, is a compound as described above, but wherein Z is hydrogen.

In other embodiments, this invention relates to the above pharmaceutical compositions for the treatment of alcoholism or alcohol dependence, and the above methods of treating alcoholism or alcohol dependence, wherein the compound of formula I, or pharmaceutically acceptable salt thereof, is a compound as described above, wherein R³ and R⁴ are independently selected from hydrogen and unsubstituted (C₁-C₄) alkyl.

In other preferred embodiments, this invention relates to the above pharmaceutical compositions for the treatment of alcoholism or alcohol dependence, and the above methods of treating alcoholism or alcohol dependence, wherein the compound of formula **I**, or pharmaceutically acceptable salt thereof, is a compound wherein one or both of R³ and R⁴ are hydrogen.

In other embodiments, this invention relates to the above pharmaceutical compositions for the treatment of alcoholism or alcohol dependence, and the above methods of treating alcoholism or alcohol dependence, wherein the compound of formula I, or pharmaceutically acceptable salt thereof, is a compound wherein R¹ and R² are independently selected from hydrogen and unsubstituted (C₁-C₄)alkyl.

In other preferred embodiments, this invention relates to the above pharmaceutical compositions for the treatment of alcoholism or alcohol dependence, and the above methods of treating alcoholism or alcohol dependence, wherein the compound of formula I, or pharmaceutically acceptable salt thereof, is a compound wherein one of R¹ and R² is hydrogen and the other of R¹ and R² is (C₁-C₄)alkyl.

In more preferred embodiments, this invention relates to the above pharmaceutical compositions for the treatment of alcoholism or alcohol dependence, and the above methods of treating alcoholism or alcohol dependence, wherein the compound of formula I, or pharmaceutically acceptable salt thereof, is a compound wherein one of R¹ and R² is hydrogen and the other of R¹ and R² is methyl.

Other preferred embodiments of this invention relate to the above pharmaceutical compositions for the treatment of alcoholism or alcohol dependence, and the above methods of treating alcoholism or alcohol dependence, wherein the compound of formula I, or pharmaceutically acceptable salt thereof, is selected from the following compounds and their pharmaceutically acceptable salts:
[4-(3,4-Dichlorophenoxy)-biphenyl-3-ylmethyl]-methylamine;
[2-(3,4-Dichlorophenoxy)-5-thiophen-3-ylbenzyl]-methylamine;
[2-(3,4-Dichlorophenoxy)-4-thiophen-3-ylbenzyl]-methylamine;
[2-(3,4-Dichlorophenoxy)-4-furan-2-ylbenzyl]-methylamine;
[2-(3,4-Dichlorophenoxy)-5-furan-2-ylbenzyl]-methylamine;
N-[4'-(3,4-Dichlorphenoxy)-3'-methylaminomethyl-biphenyl-3-yl]-acetamide;
[2-(3,4-Dichlorophenoxy)-5-thiophen-2-ylbenzyl]-methylamine;
[4-(3,4-Dichlorophenoxy)-4'-fluoro-biphenyl-3-ylmethyl]-methyamine;
[2-(3,4-Dichlorophenoxy)-5-[1,2,3]triazol-1-ylbenzyl]-methylamine;
[2-(3,4-Dichlorophenoxy)-5-[1,2,3]triazol-2-ylbenzyl]-methylamine;
[2-(3,4-Dichlorophenoxy)-5-pyridin-2-ylbenzyl]-methylamine;
[2-(3,4-Dichlorophenoxy)-5-pyridin-3-ylbenzyl]-methylamine;
1-[4-(3,4-Dichlorophenoxy)-3-methylaminomethylphenyl]-1H-pyrazol-3-ylamine;
[2-(3,4-Dichlorophenoxy)-5-pyridin-4-ylbenzyl]-methylamine;
[3-(3,4-Dichlorophenoxy)-biphenyl-4-ylmethyl]-methylamine;
[4-(3,4-Dichlorophenoxy)-4'-methyl-biphenyl-3-ylmethyl]-methylamine; and
[2-(3,4-Dichlorophenoxy)-4-thiophen-2-ylbenzyl]-methylamine.

Additional preferred embodiments of this invention relate to the above pharmaceutical compositions for the treatment of alcoholism or alcohol dependence, and the above methods of treating alcoholism or alcohol dependence, wherein the compound of formula I, or pharmaceutically acceptable salt thereof, is selected from the following compounds and their pharmaceutically acceptable salts:
[2-(3,4-dichlorophenoxy)-5-thiazol-2-ylbenzyl]-methylamine;
[2-(3,4-dichlorophenoxy)-5-(1 H-tetrazol-5-yl)benzyl]-methylamine;
[2-(3,4-dichlorophenoxy)-5-furan-3-ylbenzyl]-methylamine;
{1-[2-(3,4-dichlorophenoxy)-5-[1,2,3]triazol-1-ylphenyl]ethyl}-methylamine;
{1-[2-(3,4-dichlorophenoxy)-5-[1,2,3]triazol-2-ylphenyl]ethyl}-methylamine;
{1-[2-(3,4-dichlorophenoxy)-5-thiazol-2-ylphenyl]ethyl}-methylamine;
{1 -[2-(3,4-dichlorophenoxy)-4-[1,2,4]triazol-1 -ylphenyl]ethyl}-methylamine;
[2-(3,4-dichlorophenoxy)-5-(5-methylthiophen-2-yl)benzyl]-methylamine;
[2-(3,4-dichlorophenoxy)-5-[1,2,4]triazol-4-ylbenzyl]-methylamine;
1-[4-(3,4-dichlorophenoxy)-3-(methylaminomethyl)phenyl]-pyrrolidin-2-one;
1-[4-(3,4-dichlorophenoxy)-3-(1-methylaminoethyl)phenyl]-pyrrolidin-2-one; and
1-[4-(3,4-dichlorophenoxy)-3-(methylaminomethyl)phenyl]-piperidin-2-one.

Other embodiments of this invention relate to the above pharmaceutical compositions for the treatment of alcoholism or alcohol dependence, and the above methods of treating alcoholism or alcohol dependence, wherein the compound of formula I, or pharmaceutically acceptable salt thereof, is selected from the following compounds and their pharmaceutically acceptable salts:
[2-(3,4-dichlorophenoxy)-5-pyrimidin-2-ylbenzyl]-methylamine;
[2-(3,4-dichlorophenoxy)-5-pyrimidin-4-ylbenzyl]-methylamine;
[2-(3,4-dichlorophenoxy)-5-(2-methylpyrimidin-4-yl)-benzyl]-methylamine;
{1-[2-(3,4-dichlorophenoxy)-5-(2-methylpyrimidin-2-yl)-phenyl]-ethyl}-methylamine;
4-[4-(3,4-dichlorophenoxy)-3-(1-methylpyrrolidin-2-yl)-phenyl]-2-methylpyrimidine;
[2-(4-chlorophenoxy)-5-(1-methyl-1H-pyrrol-3-yl)-benzyl]-dimethylamine;
[5-(1-methyl-1H-pyrrol-3-yl)-2-(naphthalen-2-yloxy)-benzyl]-dimethyl amine;
[5-imidazol-1-yl-2-(naphthalen-2-yloxy)-benzyl]-dimethylamine;
1,5,5-trimethyl-3-[3-methylaminomethyl-4-(naphthalen-2-yloxy)-phenyl]-imidazolidine-2,4-dione;
1-methyl-3-[3-methylaminomethyl-4-(naphthalen-2-yloxy)-phenyl]-imidazolidine-2,4-dione;
3-[3-methylaminomethyl-4-(naphthalen-2-yloxy)-phenyl]-thiazolidine-2,4-dione;
3-[3-methylaminomethyl-4-(naphthalen-2-yloxy)-phenyl]-oxazolidine-2,4-dione;
3-[3-methylaminomethyl-4-(naphthalen-2-yloxy)-phenyl]-oxazolidin-2-one;
3-[3-methylaminomethyl-4-(naphthalen-2-yloxy)-phenyl]-thiazolidin-2-one;
1-methyl-3-[3-methylaminomethyl-4-(naphthalen-2-yloxy)-phenyl]-imidazolidin-2-one;
1-methyl-3-[3-methylaminomethyl-4-(naphthalen-2-yloxy)-phenyl]-tetrahydro-pyrimidin-2-one;
1-[4-(3,4-dichlorophenoxy)-3-methylaminomethyl-phenyl]-3-methyl-tetrahydropyrimidin-2-one;
1-[4-(3,4-dichlorophenoxy)-3-methylaminomethyl-phenyl]-3-methylimidazolidin-2-one;
3-[4-(3,4-dichlorophenoxy)-3-methylaminomethyl-phenyl]-thiazolidin-2-one;
3-[4-(3,4-dichlorophenoxy)-3-methylaminomethyl-phenyl]-oxazolidin-2-one;
[2-(3,4-dichlorophenoxy)-5-(2-methylthiazol-4-yl)-benzyl]-methylamine;
[2-(3,4-dichlorophenoxy)-5-(2-methyloxazol-4-yl)-benzyl]-methylamine;
[2-(3,4-dichlorophenoxy)-5-(2,5-dimethyloxazol-4-yl)-benzyl]-methylamine;
[2-(3,4-dichlorophenoxy)-5-(2,5-dimethylthiazol-4-yl)-benzyl]-methylamine;
[2-(3,4-dichlorophenoxy)-5-(5-methyl-[1,2,4]thiadlazol-3-yl)-benzyl]-methylamine;
[2-(3,4-dichlorophenoxy)-5-(5-methyl-[1,2,4]oxadiazol-3-yl)-benzyl]-methylamine;
[2-(3,4-dichlorophenoxy)-5-[1,2,3]oxadiazol-4-yl-benzyl]-methylamine;
[2-(3,4-dichlorophenoxy)-5-(5-methyl-[1,2,3]thiadiazol-4-yl)-benzyl]-methylamine;
[2-(3,4-dichlorophenoxy)-5-(2,4-dimethyloxazol-5-yl)-benzyl]-methylamine;
[2-(3,4-dichlorophenoxy)-5-(2,4-dimethylthiazol-5-yl)-benzyl]-methylamine;
[2-(3,4-dichlorophenoxy)-5-[1,2,4]triazol-1-ylbenzyl]-methylamine;
[2-(3,4-dichlorophenoxy)-5-(3-methyl-[1,2,4]triazol-1-yl)-benzyl]-methylamine;
[2-(4-chlorophenoxy)-5-(3,5-dimethyl-[1,2,4]triazol-1-yl)-benzyl]-methylamine;
[2-(4-chlorophenoxy)-5-tetrazol-1-ylbenzyl]-methylamine;
[2-(4-chlorophenoxy)-5-(5-methyltetrazol-1-yl)-benzyl]-dimethylamine;
[2-(4-chlorophenoxy)-5-[1,2,4]triazol-4-ylbenzyl]-dimethylamine;
[2-(4-chlorophenoxy)-5-(1-methyl-1H-tetrazol-5-yl)-benzyl]-dimethylamine; and
{1-[2-(3,4-dichlorophenoxy)-5-(1-methyl-1H-tetrazol-5-yl)-phenyl]-ethyl}-dimethylamine.

Other preferred embodiments of the present invention relate to the above pharmaceutical compositions for the treatment of alcoholism or alcohol dependence, and the above methods of treating alcoholism or alcohol dependence, wherein the compound of formula XXX, or pharmaceutically acceptable salt thereof, is selected from the following compounds and their pharmaceutically acceptable salts:
[2-(3,4-Dichlorophenoxy)-5-fluorobenzyl]-dimethylamine;
[2-(3,4-Dichlorophenoxy)-5-fluorobenzyl]-methylamine;
[2-(3,4-Dichlorophenoxy)-5-trifluoromethylbenzyl]-dimethylamine;
N-[4-(3,4-Dichlorophenoxy)-3-dimethylaminomethylphenyl]-acetamide;
{1-[2-(3,4-Dichlorophenoxy)phenyl]-ethyl}-dimethylamine;
[2-(3,4-Dichlorophenoxy)-4-trifluoromethylbenzyl]-dimethylamine;
[2-(3,4-Dichlorophenoxy)-4-trifluoromethylbenzyl]-methylamine;
[4-Chloro-2-(3,4-dichlorophenoxy)-benzyl]-methylamine;
{1-[2-(3,4-Dichlorophenoxy)-5-fluorophenyl]-ethyl}-methylamine;
{1-[2-(3,4-Dichlorophenoxy)phenyl}-ethyl}-methylamine;
{1-[2-(4-Chlorophenoxy)phenyl]ethyl}-methylamine;
[2-(3,4-Dichlorophenoxy)-5-methoxybenzyl]-methylamine;
[2-(4-Chlorophenoxy)-5-fluorobenzyl]-methylamine;
{1-[2-(4-Chlorophenoxy)-5-fluorophenyl]-ethyl}-methylamine;
[2-(3,4-Dichlorophenoxy)-5-methylbenzyl]-dimethylamine;
[4-Bromo-2-(3,4-dichlorophenoxy)-benzyl]-methylamine;
[5-Bromo-2-(3,4-dichlorophenoxy)-benzyl]-methylamine;
[2-(3,4-Dichlorophenoxy)-4,5-dimethoxybenzyl]-methylamine;
[2-(3,4-Dichlorophenoxy)-4-methoxybenzyl]-dimethylamine;
4-(3,4-Dichlorophenoxy)-3-methylaminomethyl-benzonitrile;
[2-(3,4-Dichlorophenoxy)-4,5-dimethylbenzyl]-methylamine;
3-(3,4-Dichlorphenoxy)-4-methylaminomethyl-benzonitrile;
(+)-{1-[2-(3,4-Dichlorophenoxy)-5-fluorophenyl]-ethyl}-methylamine;
(-)-{1-[2-(3,4-Dichlorophenoxy)-5-fluorophenyl]-ethyl}-methylamine;
[2-(3,4-Dichlorophenoxy)-5-trifluoromethyl-benzyl]-methylamine;
[2-(3,4-Dichlorophenoxy)-4-methoxybenzyl]-methylamine;
[2-(4-Chloro-3-fluorophenoxy)-5-fluorobenzyl]-methylamine;
[2-(3-Chloro-4-fluorophenoxy)-5-fluorobenzyl]-methylamine;
(+/-)-2-[2-(3,4-Dichlorophenoxy)-5-fluorophenyl]-pyrrolidine;
(-)-2-[2-(3,4-Dichlorophenoxy)-5-fluorophenyl]-pyrrolidine;
(+)-2-[2-(3,4-Dichlorophenoxy)-5-fluorophenyl]-pyrrolidine; and
2-[2-(3,4-Dichlorophenoxy)-5-fluorophenyl]-N-methylpyrrolidine.

Other embodiments of this invention relate to the above pharmaceutical compositions for the treatment of alcoholism or alcohol dependence, and the above methods of treating alcoholism or alcohol dependence, wherein the compound of formula XXX, or pharmaceutically acceptable salt thereof, is selected from the following compounds and their pharmaceutically acceptable salts:
{1-[2-(3,4-Dichlorophenoxy)-5-fluorophenyl]-1-methylethyl}-methylamine;
{1-[2-(3,4-Dichlorophenoxy)-5-fluorophenyl]-1-methylethyl}-dimethylamine;
[4-Chloro-2-(4-chlorophenoxy)-5-fluorobenzyl]-methylamine;
[2-(3,4-Dichlorophenoxy)-5-fluoro-4-methoxybenzyl]-methylamine;
[4-(3,4-Dichlorophenoxy)-3-(dimethylaminomethyl)-phenyl]-dimethylamine
[5-Fluoro-2-(4-fluoro-3-methoxyphenoxy)-benzyl]-dimethylamine;
[2-(4-Chlorophenoxy)-5-isopropylbenzyl]-methylamine;
{1-[2-(4-Chlorophenoxy)-5-trifluoromethylphenyl]-ethyl}-methylamine;
[2-(4-Chlorophenoxy)-4,5-dimethylbenzyl]-methylamine;
{1-[5-Chloro-2(3,4-dichlorophenoxy)phenyl]-propyl}-methylamine;
[2-(3,4-Dichlorophenoxy)-5-methylsulfanyl-benzyl]-methylamine;
{1-[2-(3,4-Dichlorophenoxy)-5-methylsulfanyl-phenyl]-ethyl}-methylamine;
{1-[2-(3,4-Dichloro-phenoxy)-5-methylsulfanyl-phenyl]-1-methylethyl}-methylamine;
[2-(3,4-Dichlorophenoxy)-5-methylsulfanyl-benzyl]-dimethylamine;
[2-(3,4-Dichlorophenoxy)-5-methanesulfinyl-benzyl]-dimethylamine;
[2-(3,4-Dichlorophenoxy)-5-methanesulfinyl-benzyl]-methylamine;
[2-(3,4-Dichlorophenoxy)-5-methanesulfonyl-benzyl]-methylamine;
[2-(3,4-Dichlorophenoxy)-5-methanesulfonyl-benzyl]-dimethylamine;
[2-(3,4-Dichlorophenoxy)-5-(propane-2-sulfonyl)-benzyl]-methylamine;
2-[2-(3,4-Dichlorophenoxy)-5-fluorophenyl]-piperidine;
2-[2-(3,4-Dichlorophenoxy)-5-fluorophenyl]-1-methyl-piperidine;
3-[2-(3,4-Dichlor-phenoxy)-5-fluorophenyl]-4-methyl-morpholine;
2-[2-(3,4-Dichlorophenoxy)-5-fluorophenyl]-1,2-dimethyl-piperidine;
{1-[2-(3,4-Dichlorophenoxy)-5-fluorophenyl]-cyclopropyl}-dimethylamine;
2-[2-(3,4-Dichlorophenoxy)-5-fluorophenyl]-1,5-dimethyl-pyrrolidine;
3-[2-(3,4-Dichlorophenoxy)-5-fluorophenyl]-4-methyl-thiomorpholine;
{1-[2-(3,4-Dichlorophenoxy)-5-fluorophenyl]-cyclopentyl}-methylamine;
{1-[2-(3,4-Dichlorophenoxy)-5-(propane-2-sulfonyl)-phenyl]-ethyl}-methylamine; and
[4-Chloro-2-(3,4-dichlorophenoxy)-5-methanesulfonyl-benzyl]-methylamine.

In other embodiments, this invention relates to the above pharmaceutical compositions for the treatment of alcoholism or alcohol dependence, and the above methods of treating alcoholism or alcohol dependence, wherein the compound of formula XXX, or pharmaceutically acceptable salt thereof, is a compound wherein m is zero, n is one, R³ and R⁴ are hydrogen, X is chloro, bromo, iodo or methyl, R¹ is hydrogen and R² is methyl.

An example of an opioid antagonist that may be used in the methods and pharmaceutical compositions of this invention is naltrexone (17-(cyclopropylmethyl)-4,5-epoxy-3,14-dihydroxy-5α-morphinan-6-one, ReVia®, Trexan®) (also EN 1639A, UM 792, Nemexin, Trexan Du-P, Trexan DuPont, Nalorex, Antaxone, Basinal, Celupan, and Revez), as depicted below, and its pharmaceutically acceptable salts. 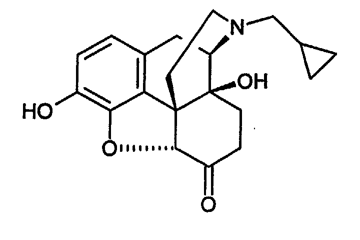 The activity and synthesis of naltrexone is referred to in United States Patent No. 3,332,950, issued July 25, 1967 (product patent), which is incorporated herein by reference in its entirety.

Another example of an opioid antagonist that may be used in the methods and pharmaceutical compositions of this invention is nalmefene (17-(cyclopropylmethyl)-4,5-epoxy-6-methylene-5α-morphinan-3,14-diol, Revex®) (also Nalmetrene, JF 1, Incystene, Arthene, Fenarc and Cervene®), as depicted below, and its pharmaceutically acceptable salts. 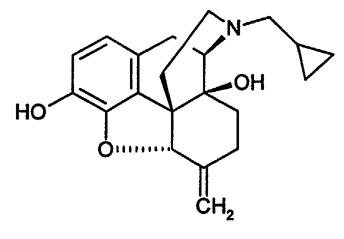 The activity and synthesis of nalmefene is referred to in United States Patent No. 3,814,768, issued June 4, 1974 (product patent) and United States Patent No. 3,896,226, issued July 22, 1975 (composition patent), all of which are incorporated herein by reference in their entirety.

Another example of an opioid antagonist that may be used in the methods and pharmaceutical compositions of this invention is nalorphine (7,8-didehydro-4,5-epoxy-17-(2-propenyl)-morphinan-3,6-diol, Miromorfalil), as depicted below, and its pharmaceutically acceptable salts. 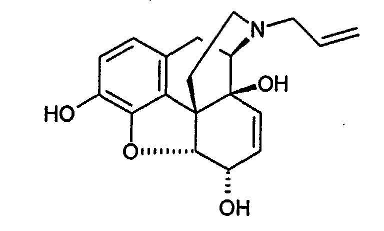 The activity and synthesis of nalorphine is referred to in United States Patent Nos. 2,364,833 and 2,891,954, issued December 12, 1944 and June 23, 1959, respectively, which are incorporated herein by reference in their entirety.

Another example of an opioid antagonist that may be used in the methods and pharmaceutical compositions of this invention is naloxone (4,5-epoxy-3,14-dihydroxy-17-(2-propenyl)-morphinan-6-one, Narcan®) (also naloxone hydrochloride), as depicted below, and its pharmaceutically acceptable salts. 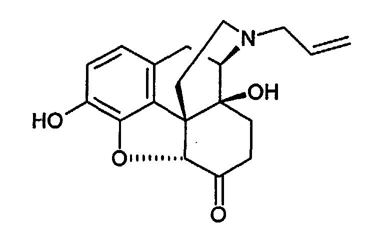 The activity and synthesis of naloxone is referred to in United States Patent No. 3,254,088, issued May 31, 1966, which is incorporated herein by reference in its entirety.

Another example of an opioid antagonist that may be used in the methods and pharmaceutical compositions of this invention is naltriben (17-(cyclopropylmethyl)-6,7-didehydro-3,14β-dihydroxy-4,5a-epoxy-6,7-2',3'-benzo[b]furanomorphinan), as depicted below, and its pharmaceutically acceptable salts. 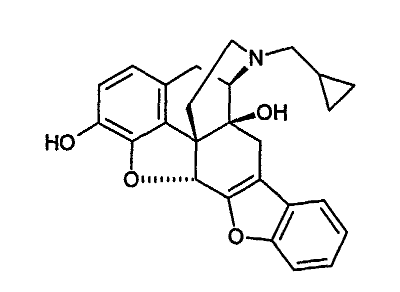 The activity and synthesis of naltriben is referred to in United States Patent No. 4,816,586, issued March 28, 1989, which is incorporated herein by reference in its entirety.

Another example of an opioid antagonist that may be used in the methods and pharmaceutical compositions of this invention is naltrindole (17-(cyclopropylmethyl)-6,7-dehydro-4,5α-epoxy-3,14-dihydroxy-6,7-2',3'-indolomorphinan, NTI®), as depicted below, and its pharmaceutically acceptable salts. 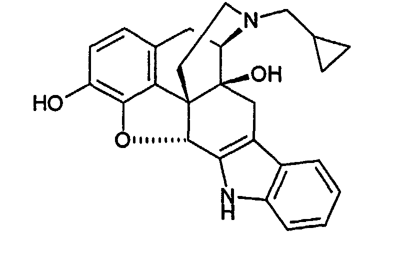 The activity and synthesis of naltrindole is referred to in United States Patent No. 4,816,586, issued March 28, 1989, which is incorporated herein by reference in its entirety.

Another example of an opioid antagonist that may be used in the methods and pharmaceutical compositions of this invention is cyprodime ((-)-N-(cyclopropylmethyl)-4,14-dimethoxy-morphinan-6-one), as depicted below, and its pharmaceutically acceptable salts. 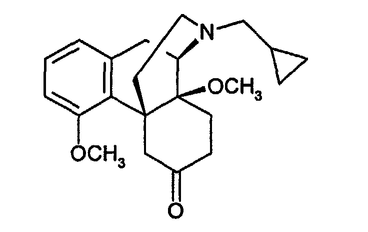 The activity and synthesis of cyprodime is referred to in International Patent Application No. WO 93/02707, published February 18, 1993, which is incorporated herein by reference in its entirety. The activity of cyprodime is also referred to in Helmut Schmidhammer, *et al*., "Synthesis and Biological Evaluation of 14-alkoxymorphinans. 2. (-)-N-(cyclopropylmethyl)-4,14-dimethoxymorphinan-6-one, a selective µ opioid receptor antagonist," Journal of Medicinal Chemistry, Vol. 32, p. 418-421 (1989).

Another example of an opioid antagonist that may be used in the methods and pharmaceutical compositions of this invention is DPI-2505 ([3a,4(Z),5a]-4-[[4-(2-butenyl)-3,5-dimethyl-1-piperazinyl](3-hydroxyphenyl)methyl]-N,N-diethylbenzamide monohydrochloride), as depicted below, and its pharmaceutically acceptable salts. 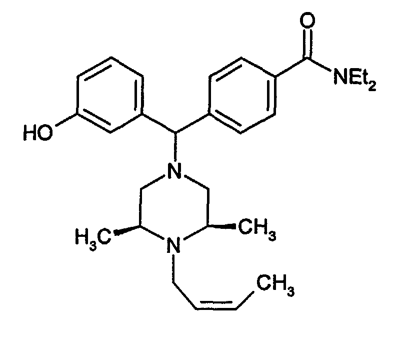 The activity and synthesis of DPI-2505 is referred to in United States Patent No. 5,658,908, issued August 19, 1997, and in corresponding International Patent Application No. WO 93/15062, published August 5, 1993, all of which are incorporated herein by reference in their entirety.

Other examples of opioid antagonists that may be used in the methods and pharmaceutical compositions of this invention include compounds disclosed in United States Patent Application No. 09/467,871, filed December 20, 1999, allowed April 20, 2001, and in counterpart International Patent Application No. WO 00/39089, published July 6, 2000, and their pharmaceutically acceptable salts, the activity and synthesis of which is referred to in the aforementioned patent applications, which applications are incorporated herein by reference in their entirety.

Other examples of opioid antagonists that may be used in the methods and pharmaceutical compositions of this invention include compounds disclosed in United States Patent Application No. 09/323,332, filed June 1, 1999, and in counterpart International Patent Application No. WO 00/14066, published March 16, 2000, and their pharmaceutically acceptable salts, the activity and synthesis of which is referred to in the aforementioned patent applications, which applications are incorporated herein by reference in their entirety.

Other examples of opioid antagonists that may be used in the methods and pharmaceutical compositions of this invention include compounds disclosed in United States Patent Application No. 09/369,841, filed August 6, 1999, and in counterpart International Patent Application No. WO 00/39091, published July 6, 2000, and their pharmaceutically acceptable salts, the activity and synthesis of which is referred to in the aforementioned patent applications, which applications are incorporated herein by reference in their entirety.

Other examples of opioid antagonists that may be used in the methods and pharmaceutical compositions of this invention include compounds disclosed in United States Patent Application No. 09/503,679, filed February 14, 2000, and their pharmaceutically acceptable salts, the activity and synthesis of which is referred to in the aforementioned patent application, which application is incorporated herein by reference in its entirety.

Other examples of opioid antagonists that may be used in the methods and pharmaceutical compositions of this invention include compounds disclosed in United States Provisional Patent Application No. 60/218,500, filed July 14, 2000, and in counterpart United Kingdom Patent Application No. (GB) 0015562.2, filed June 23, 2000, and their pharmaceutically acceptable salts, the activity and synthesis of which is referred to in the aforementioned patent applications, which applications are incorporated herein by reference in their entirety.

The term "treating", as used herein, refers to reversing, alleviating, or inhibiting the progress of the disease, disorder or condition, or one or more symptoms of such disease, disorder or condition, to which such term applies. Depending on the condition of the patient, as used herein, this term also refers to preventing a disease, disorder or condition, and includes preventing the onset of a disease, disorder or condition, or preventing the symptoms associated with a disease, disorder or condition. As used herein, this term also refers to reducing the severity of a disease, disorder or condition or symptoms associated with such disease, disorder or condition prior to affliction with the disease, disorder or condition. Such prevention or reduction of the severity of a disease, disorder or condition prior to affliction refers to administration of the composition of the present invention, as described herein, to a subject that is not at the time of administration afflicted with the disease, disorder or condition. "Preventing" also refers to preventing the recurrence of a disease, disorder or condition or of symptoms associated with a disease, disorder or condition. The terms "treatment" and "therapeutically" refer to the act of treating, as defined above.

The term "mammal", as used herein, refers to any member of the class "Mammalia", including, but not limited to, humans, dogs and cats.

The pharmaceutical compositions and methods of this invention comprise, or comprise administering, monoamine reuptake inhibitors of formulas I and XXX through XXXII, which may have chiral centers and therefore exist in different enantiomeric forms. This invention includes methods and pharmaceutical compositions, as described above, wherein the monoamine reuptake inhibitors (*i.e*., the compounds of formulas I, XXX, XXXI and XXXII) that are employed are optical isomers, tautomers or stereoisomers of the compounds of formulas I and XXX through XXXII, as defined above, or mixtures thereof.

Those monoamine reuptake inhibitors of formulas I and XXX through XXXII that contain basic groups can form acid addition salts with various inorganic and organic acids. The present invention also relates to pharmaceutical compositions and methods comprising, or comprising administering, pharmaceutically acceptable acid addition salts of monoamine reuptake inhibitors of formulas I and XXX through XXXII and of opioid antagonists. The chemical acids that may be used as reagents to prepare the pharmaceutically acceptable acid salts of the basic active agents employed in the methods and pharmaceutical compositions of this invention are those that form non-toxic acid salts with such compounds. Such non-toxic acid salts include, but are not limited to, those derived from salts containing pharmacologically acceptable anions, such as the hydrochloride, hydrobromide, hydroiodide, nitrate, sulfate, bisulfate, phosphate, acid phosphate, acetate, lactate, citrate, acid citrate, tartrate, bitartrate, succinate, maleate, fumarate, gluconate, saccharate, benzoate, methanesulfonate, ethanesulfonate, benzenesulfonate, p-toluenesulfonate and pamoate [*i.e*., 1,1'-methylene-bis-(2-hydroxy-3- naphthoate)]salts.

Those monoamine reuptake inhibitors of formulas I and XXX through XXXII that contain acidic groups can form base addition salts with certain bases. The present invention also relates to pharmaceutical compositions and methods comprising, or comprising administering, pharmaceutically acceptable base addition salts of monoamine reuptake inhibitors of formulas I and XXX through XXXII and of opioid antagonists. The chemical bases that may be used as reagents to prepare the pharmaceutically acceptable base salts of the acidic active agents that are employed in the methods and pharmaceutical compositions of this invention are those that form non-toxic base salts with such compounds. Such non-toxic base salts include, but are not limited to, those derived from such pharmacologically acceptable cations such as alkali metal cations *(e.g.,* potassium and sodium) and alkaline earth metal cations *(e.g.,* calcium and magnesium), ammonium or water-soluble amine addition salts such as N-methylglucamine (meglumine), and the lower alkanolammonium and other base salts of pharmaceutically acceptable organic amines.

The present invention also relates to pharmaceutical compositions and methods of treatment that employ isotopically-labeled compounds that are identical to those recited in formulas I and XXX through XXXII but for the fact that one or more atoms are replaced by an atom having an atomic mass or mass number different from the atomic mass or mass number usually found in nature. Examples of isotopes that can be incorporated into the monoamine reuptake inhibitors of formulas I and XXX through XXXII that are employed in the pharmaceutical compositions and methods of the present invention include isotopes of hydrogen, carbon, nitrogen, oxygen, phosphorous, sulfur, fluorine and chlorine, such as ²H, ³H, ¹³C, ¹¹C, ¹⁴C, ¹⁵N, ¹⁸O, ¹⁷O, ³¹P, ³²P, ³⁵S, ¹⁸F, and ³⁶Cl, respectively. The monoamine reuptake inhibitors of formulas I and XXX through XXXII employed in the pharmaceutical compositions and methods of the present invention, prodrugs thereof, and pharmaceutically acceptable salts of such compounds or of such prodrugs which contain the aforementioned isotopes and/or other isotopes are within the scope of this invention. Such compounds may be useful as research and diagnostic tools in metabolism pharmacokinetic studies and in binding assays. Certain isotopically-labeled monoamine reuptake inhibitors of formulas I and XXX through XXXII, for example, those into which radioactive isotopes such as ³H and ¹⁴C are incorporated, are useful in drug and/or substrate tissue distribution assays. Tritiated, *i.e*., ³H, and carbon-14, *i.e.,* ¹⁴C, isotopes are particularly preferred for their ease of preparation and detectability. Further, substitution with heavier isotopes such as deuterium, *i.e*., ²H, can afford certain therapeutic advantages resulting from greater metabolic stability, for example increased *in vivo* half-life or reduced dosage requirements and, hence, may be preferred in some circumstances. Isotopically-labeled monoamine reuptake inhibitors of formulas I and XXX through XXXII and prodrugs thereof can generally be prepared by carrying out the procedures disclosed in the schemes and discussion of the schemes below and substituting a readily available isotopically-labeled reagent for a nonisotopically-labeled reagent.

### Detailed Description Of The Invention

In the discussion that follows, formulas I and XXX through XXXII are defined as set forth above in the Summary of the Invention.

Compounds of the formula I and their pharmaceutically acceptable salts can be prepared as described below and in U.S. Patent Application No. 09/692,335, filed October 19, 2000, entitled "Novel Biaryl Ether Derivatives Useful As Monoamine Reuptake Inhibitors," and International Patent Application No. PCT/IB00/01373, filed September 25, 2000, each claiming priority from U.S. Provisional Application No. 60/167,761, filed November 29, 1999. The foregoing patent applications are incorporated herein by reference in their entirety.

Schemes **1-5** below and the discussion of Schemes **1-5** that follows illustrate methods of preparing compounds of the formula I. Unless otherwise indicated, in Schemes **1-5** and the discussion of Schemes **1-5** that follows, A, B, R¹, R², R³, R⁴, R⁵, R⁶, X, Y, Z, m, n and p are as defined above for compounds of the formula I.

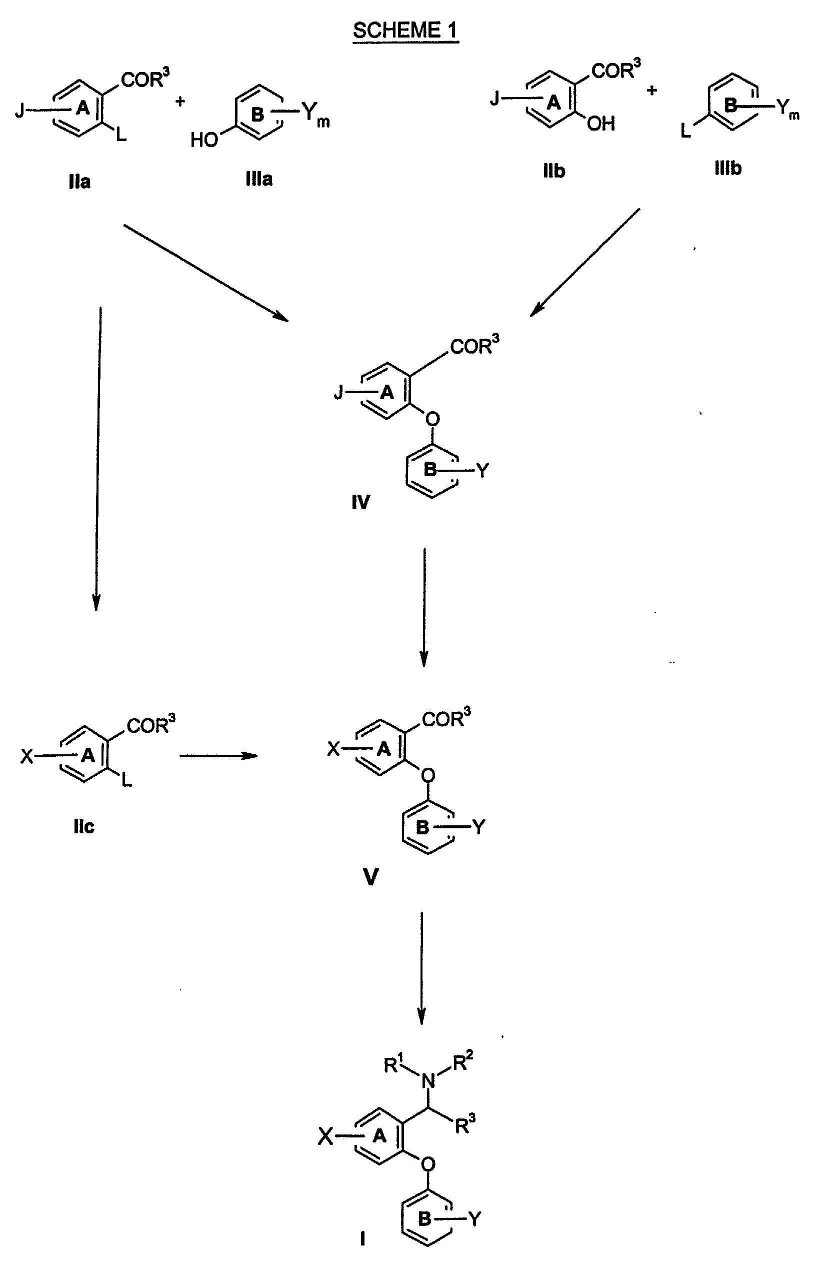

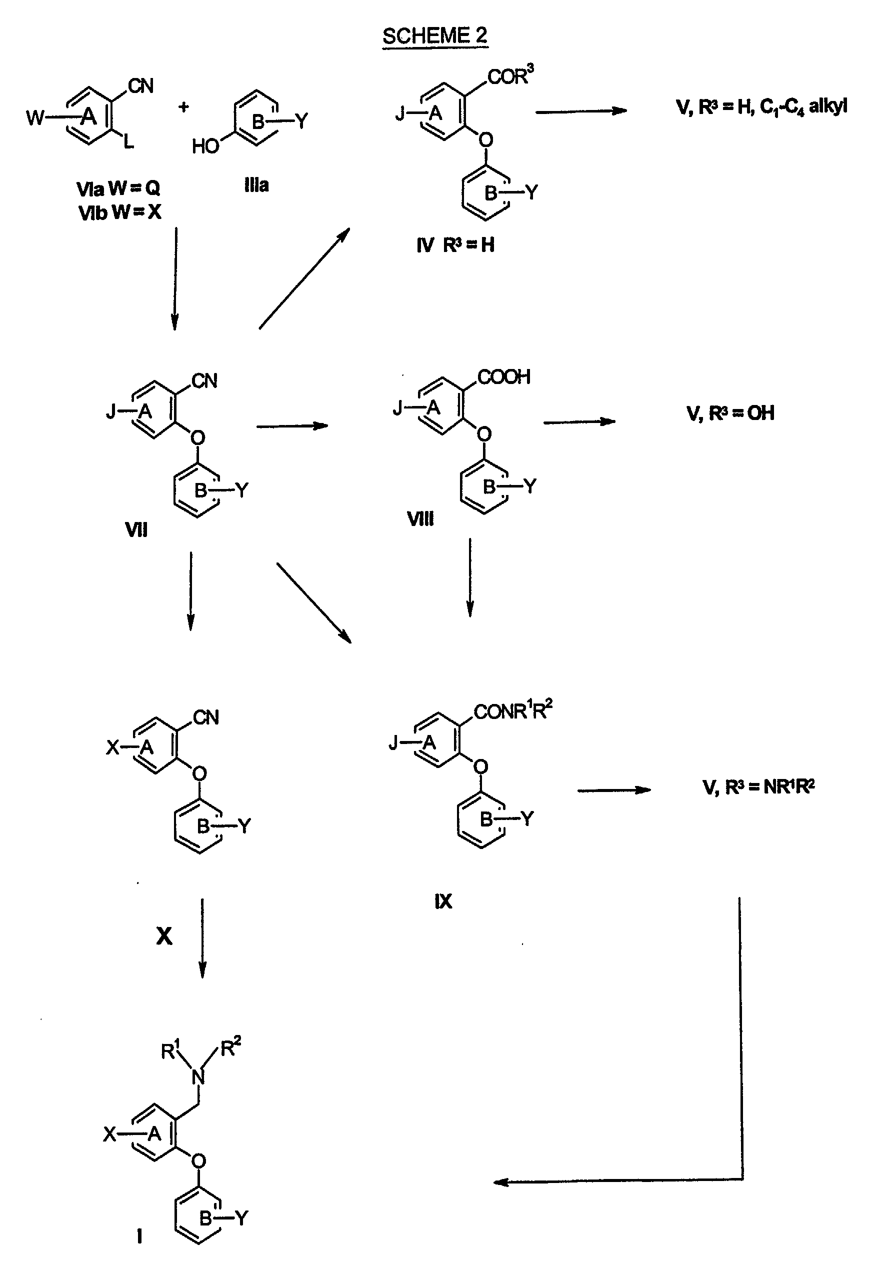

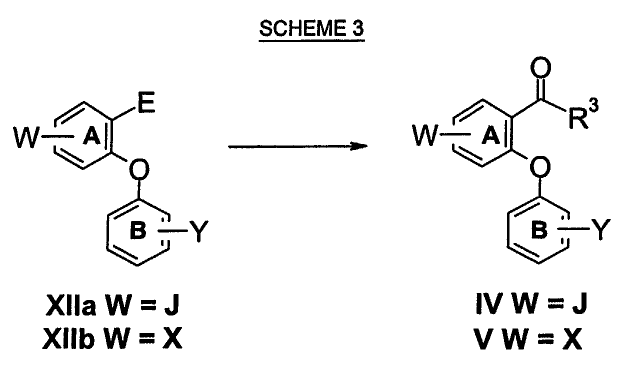

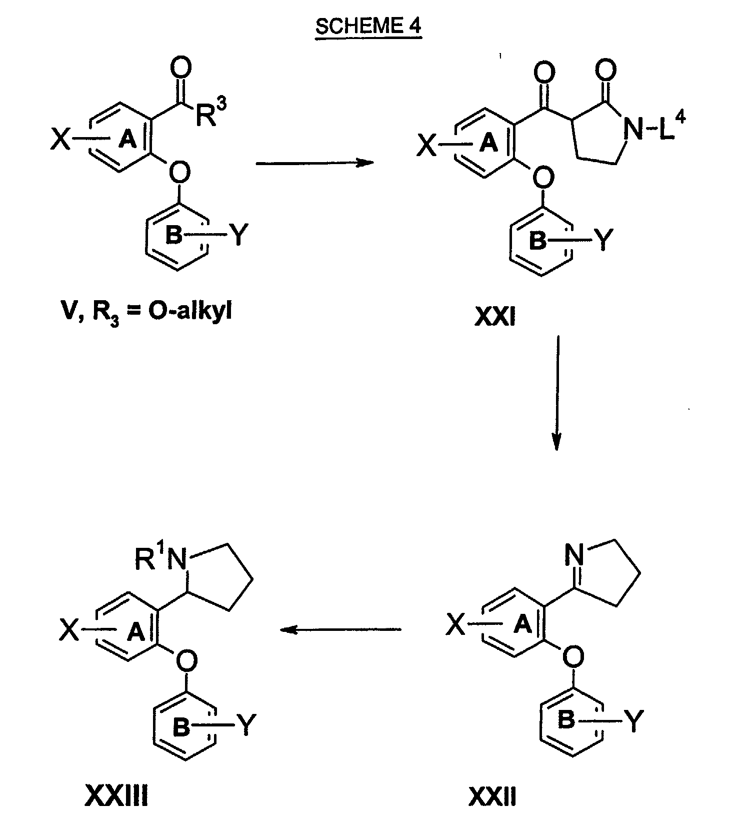

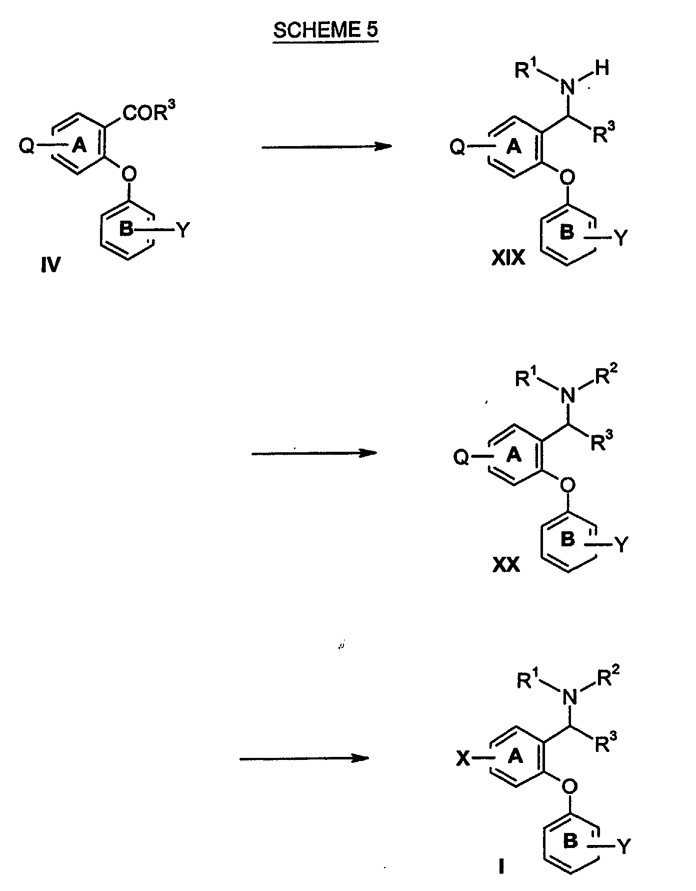

Scheme 1 refers to the preparation of compounds of the formula I from compounds of the formulae **II** and **III**. L represents a suitable leaving group such as fluoro, chloro, nitro, or triflate. In scheme 1, Z is hydrogen. However, using the appropriate starting compound of formula **II**, compounds of formula **I** wherein Z is other than hydrogen can be prepared according to the same scheme. Compounds of the formulas **IIa, IIb, IIIa** and **IIIb** are commercially available or can be made by methods well known to those of ordinary skill in the art. For example, compounds of general formulas **IIa** and **IIb** wherein R³ is H may be prepared by introducing an aldehyde functional group (CHO) to a compound of formula **XV** or **XVI,** respectively, using methods well known to those of skill in the art.

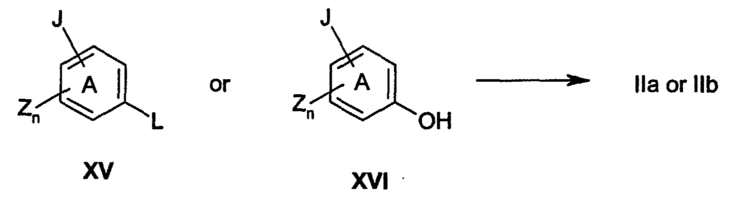

When L = F, the procedure of A. J. Bridges et al., Tetrahedron Letters, **1992,** 33(49), 7499-7502, is particularly useful for the synthesis of substituted ortho-fluorobenzaldehydes. Other such transformations have been described by C. F. H. Allen et al., Organic Synthesis, **1951**, 31, 92; T. DePaulis et al, Journal of Medicinal Chemistry, **1986,** 29, 61; I. M. Godfrey et al., J. Chemical Society, Perkin Transactions 1, **1974,** 1353; K. M. Tramposil et al., Journal of Medicinal Chemistry, **1983,** 26(2), 121; and M. E. Cracknell et al., Chemistry and Industry, (London), **1985,** (9), 309.

Referring to Scheme 1, a compound (i.e., an aldehyde or ketone) of the formula **IIa** is reacted with a compound (i.e., a phenol) of the formula **IIIa** in the presence of a base to form the corresponding compound of formula **IV.** This reaction is generally carried out at a temperature from about 0°C to about 150°C for about 1 hour to about 3 days, preferably at about 90-95°C for about 18 hours, in a polar solvent such as dimethyl sulfoxide (DMSO), N,N-dimethylformamide (DMF), N,N-dimethylacetamide (DMA) or N-methyl-2-pyrrolidinone (NMP), preferably DMF. Suitable bases include anhydrous sodium carbonate (Na₂CO₃), potassium carbonate (K₂CO₃), sodium hydroxide (NaOH), potassium hydroxide (KOH) and amines such as pyrrolidine, triethylamine and pyridine, with anhydrous K₂CO₃ being preferred. Details for conducting this procedure can be found in G. W. Yeager et al., Synthesis, **1995,** 28-30; J. R. Dimmock et al., Journal of Medicinal Chemistry, **1996,** 39(20), 3984-3997. Alternatively, phenols of the formula **IIb** and compounds of the formula **IIIb** may be converted into aldehydes or ketones of the general formulae **IV** according to the procedures described by K. Tomisawa et al., Chemical and Pharmaceutical Bulletin, **1984,** 32(8), 3066-3074.

Next, a compound of the formula **IV**, wherein J is a leaving group, for example bromine, iodine, triflate, fluorosulfonate or methanesulfonate, can be converted to a compound of the formula **V** by reaction with a compound of the general formula **X-G,** wherein G is defined as a reactive leaving group such as B(OH)₂, Sn[(C₁-C₆)alkyl], Zn(Hal) and the like, usually in the presence of a catalytic amount of a catalyst, e.g., tetrakis(triphenylphosphine) palladium(0), tetrakis(triphenylphosphine) nickel(0) or dichlorobis(triphenylphosphine) palladium(II), among others, and in the presence of a base such as sodium carbonate, potassium carbonate or triethylamine. The reactions can be conducted in a variety of organic solvents (e.g., benzene, dimethoxyethane) or in mixtures such as aqueous N,N-dimethylformamide or aqueous ethanol at temperatures in the range of about 0 °C to about 100°C. A good general reference for this process may be found in the review by Stephen Stanforth, Tetrahedron, **1998,** 54, 263-303. Other specific references include M. J. Sharp et al, Synthetic Communications, **1981,** 11(7), 513; R. B. Miller et al, Tetrahedron Letters, **1989,** 30(3), 297; W. J. Thompson et al, Journal of Organic Chemistry, **1984,** 49(26), 5237. The compounds of the general formula **X-G** are in many cases commercially available or can be prepared by one skilled in the art of organic synthesis (for example, see the procedures in M.J. Sharp and V. Snieckus, Tetrahedron Letters, **1985,** 26(49), 5997-6000; G. W. Kabalka et al, Journal of Organometallic Chemistry, **1983,** 259, 269-274).

Alternatively, an intermediate of the formula **IIa** may be converted into a compound of the formula **llc,** wherein X is as defined above, using the methods described above for the conversion of compounds of formula **IV** to **V.** These intermediates of formula **IIc** can then be reacted with a compound of the general formula **IIIa** to produce the ethers of general formula **V** using the methods described above for the conversion of compounds of formula **IIa** to **IV.**

Additionally, compounds of formulae **IIa** or **IV,** wherein J is a functional group like CN, can be converted to compounds of the formula **IIc** or **V** wherein X is a moiety such as 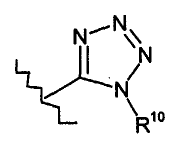 and wherein R¹⁰ is independently chosen from hydrogen, (C₁-C₆)alkyl, aryl-(C₁-C₆)alkyl or aryl, optionally substituted with hydrogen, halo, (C₁-C₆)alkyl, (C₁-C₆)alkoxyl or (C₁-C₆)SOᵣ, where r is zero, one or two. Methods for this conversion are well documented in the chemical literature; for example, through the use of sodium azide and lithium chloride in 2-methoxyethanol according to the procedure described by J. Sauer et al, Tetrahedron, **1960**, 11, 241. Under these conditions, it may be necessary to initially protect the COR³ group of compound **IIa** or **IV** to effectively convert the J group to the corresponding group X of compounds **IIc** or **V,** respectively. There are many protecting groups available which can be utilized in this process, including acetals and ketals which are described and referenced by P. G. M. Wuts and T. W. Green in Protective Groups in Organic Synthesis, 2^{nd} ed., John Wiley and Sons, New York, **1991,** pp 175-223. The selection of an appropriate protecting group can be made based upon the presence of other reactive groups in the molecule.

Subsequently, compounds of the formula **V** (R³ = H or (C₁-C₄)alkyl) can be converted into compounds of the formula **I** by subjecting them to reductive amination conditions. For example, a compound of the formula **V** can be reacted with a compound of the formula HNR¹R² to form an intermediate of the formula **XVII:** 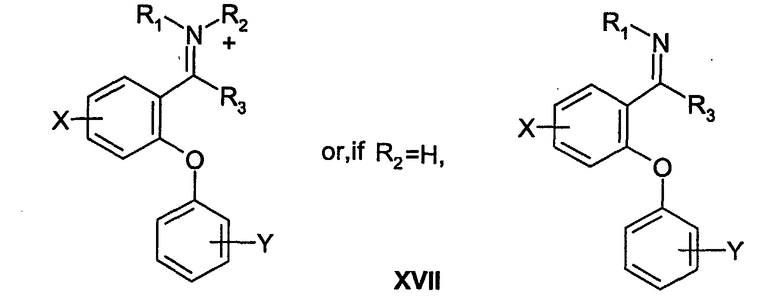 which may be isolated or converted directly in the same reaction step into a compound of the formula **I.** This conversion, whether in situ or starting with the isolated compound of formula **XVII,** can be performed using one or more methods known to those skilled in the art.

For example, the compound of formula **V** and the appropriate compound of formula HNR¹R² can be combined in the presence of a dehydrating reagent such as titanium (IV) tetrachloride or titanium (IV) isopropoxide, in a reaction inert solvent such as benzene, toluene, ethanol or a like solvent, until the reaction is judged to be complete, according to the procedure of S. Bhattarcharyya (Journal of Organic Chemistry, **1995,** 60(15), 4928-4929). Alternatively, the compound of formula **V** and the compound of formula HNR¹R² can be combined in an inert solvent such as benzene or toluene, in the presence or absence of a water scavenger such as molecular sieves, and heated to eliminate water generated during the formation of the intermediate of formula **XVII.** The degree of completion of the conversion of compounds of the formula **IV** into the above intermediate(s) of formula **XVII** can be assessed using one or more known analytical techniques, including ¹H-NMR spectroscopy.

In some instances, it may be possible or desirable to isolate the intermediate(s) of formula **XVII**, or they may be further reacted with a reducing agent selective for the reduction of the intermediate to the desired compounds of formula **I.** Such reducing agents are widely known to those skilled in the art and include, for example, sodium borohydride (NaBH₄), sodium cyanoborohydride (NaBH₃CN) and sodium triacetoxy-borohydride (NaBH(OAc)₃), as described by A. F. Abdel-Magid et al. in Tetrahedron Letters, **1990,** 31, 5595. This reduction is generally carried out in a polar solvent such as methanol, ethanol, isopropanol or a like solvent, and at temperatures of about 0°C to about 100°C, preferably at room temperature. In the procedure described by Bhattarcharyya, the intermediate of formula **XVII** is formed in an ethanol solvent and, without isolation, is reduced to the product of formula **I** using NaBH₄.

As an alternative to the aldehyde or ketone intermediates of formulae **IV** and **V,** one skilled in the art can also prepare compounds of formula **VII** (i.e., nitriles), beginning with compounds of the formulae **IIIa** and **VI,** as illustrated in Scheme 2, using the diphenyl ether formation procedure described in Scheme 1. These compounds can then serve as intermediates for the syntheses of the desired compounds of formula **I.** Procedures for preparation of the compounds of formula **VI** used in this process can be adapted from those found in the literature. (See, e.g., D. C. Remy et al., Journal of Medicinal Chemistry, **1975**, 18(2), 142-148; E. A. Schmittling et al., Journal of Organic Chemistry, **1993**, 58(12), 3229-3230).

The conversion of the nitriles of formula **VII** so obtained into the desired products of formula **I** can be achieved by several routes, as depicted in Scheme 2. For example, the nitrile group of **VII** can be hydrolyzed under acidic conditions using methods well known to those of skill in the art, to produce a carboxylic acid derivative of formula **VIII.** (See, e.g., A. I. Meyers et al., Tetrahedron Letters, **1984,** 25 (28), 2941; and R. W. Higgins et al., Journal of Organic Chemistry, **1951**, 16, 1275). This carboxylic acid derivative can then be converted to a compound of the formula **V** (R³ = OH), using procedures previously described in Scheme 1 for the conversion of **IV** to **V;** subsequently compound **V** (R³ = OH) can be converted to compound **V** (R³ = NR¹R²) and then to the compounds of formula **I** as described below.

Alternatively, compound **VIII** can be converted into a carboxamide derivative of formula **IX** using one or more standard methods which are disclosed in the chemical literature, e.g., via reaction of an acid halide prepared from a compound of the formula **VIII** with an amine of general formula HNR¹R² (see R. E. Kent et al., Organic Synthesis, Coll. Vol. III, 1955, 490; and R. M. Herbst et al., Organic Synthesis, Coll, Vol. II, **1943**, 11 for discussions of the Schotten-Bauman reaction). These carboxamides of formula **IX** can then be converted to the corresponding carboxamides of formula **V** (R³ = NR¹R²) by replacing the J substituent with the appropriate X group using conditions similar to those described for converting **IV** to **V** in Scheme 1.

The carboxamides of formulae **V** so prepared can then be reduced to the final products of formulae I using an appropriate reduction process. Depending on the presence of substituents X, Y and Z on the carboxamides **V**, this reduction can be accomplished using one or more of a variety of reagents including lithium aluminum chloride (e.g., J. Lehmann et al, Archiv. Pharm. (Weinheim, Ger.), **1982,** 315 (11), 967; N. S. Narasimhan and P. A. Patil, Journal of the Chemical Society, Chemical Communications, **1987,** (3), 191), diborane (H. C. Brown et al, Journal of the American Chemical Society, **1970,** 92, 1637 and **1973,** 38, 912; N. M. Moon et al, Journal of Organic Chemistry, **1973,** 38, 2786; H. C. Brown and V. Verma, Journal of Organic Chemistry, **1974,** 39, 1631), thexylborane / diethylaniline (A. Pelter et al, Tetrahedron Letters, **1978,** 4715), phosphorus trichloride / toluene followed by ethanolic sodium borohydride (A. Rahman et al, Tetrahedron Letters, **1976,** 219) or aluminum hydride (H. C. Brown et al, Journal of the American Chemical Society, **1966,** 88,1464; A. F. Burchat et al, Journal of Organic Chemistry, 1996, 61(21), 7627-7630).

The resulting carboxamides of the formula **IX,** wherein R¹ and R² are hydrogen, can also be prepared directly from the corresponding nitriles of formula **VII** by specific hydrolysis methods, employing, for example, hydrogen peroxide or strong aqueous alkali metal salts. (See Chemistry & Industry, **1961,** 1987; C. R. Noller, Organic Synthesis, Coll. Vol. II, **1943,** 586; and J. H. Hall and M. Gisler, Journal of Organic Chemistry, **1976,** 41, 3769). Subsequently, the carboxamide derivatives of formula **IX** may can be converted to the carboxamide compounds of formula **V** (R³ = NR¹R²) in the manner just described for the conversion of **VIII** to **V**.

Finally, the nitriles of formula **X,** obtained from the nitriles of formula **VII** analogously to the conversion of compounds of formulae **IV** to **V,** can be reduced to the desired compounds of general formula **I**, wherein R¹ and R² are both hydrogen, by using one of a variety of reducing agents disclosed in the chemical literature which are selective for this transformation (including catalytic hydrogenation using hydrogen gas and platinum (II) oxide, as described by J. A. Secrist, III and M. W. Logue in Journal of Organic Chemistry, **1972,** 37, 335; hydrazine hydrate and Raney nickel in ethanol, as described by W. W. Zajac, Jr. et al. in Journal of Organic Chemistry, **1971,** 36, 3539; and sodium trifluoroacetoxy borohydride in THF, as described by N. Umino et al. in Tetrahedron Letters, **1976,** 2875). Such reducing agents can also include lithium aluminum hydride in a nonreactive solvent such as diethyl ether or tetrahydrofuran (see, e.g., A. C. Cope et al., Organic Synthesis, Coll. Vol. IV, **1963,** 339, for use of lithium aluminum hydride in a diethyl ether or THF solvent).

The nitriles of formula **VII** may also be converted to the corresponding aldehydes of general formula **IV,** wherein R³ is hydrogen, using reagents and conditions which are specific for this transformation, such as lithium triethoxyaluminum hydride in a solvent such as THF or diethyl ether, as described by H. C. Brown and C. P. Garg in Journal of the American Chemical Society, **1964,** 86, 1085 and by J. Malek and M. Cerny in Synthesis, **1972,** 217.

In addition to the methods described above in Schemes 1 and 2 for the preparation of the intermediate aldehydes and ketones of formula **I**, other methods exist which can provide compounds of the formula **I.** For example, in the procedure depicted in Scheme 3, a compound of formula **XIIa,b,** in which E is a hydrogen atom, can be reacted, under conditions of Friedel-Crafts acylation (e.g., AlCl₃/CH₂Cl₂/R³COCl), to produce ketones of the formula **IV** or **V** in which R³ is C₁-C₄ alkyl (C. F. H. Allen, Organic Synthesis, Coll. Vol. II, 3, **1943**). Alternatively, an acid anhydride, i.e., (R³CO)₂O can be reacted under similar conditions (O. Grummitt et al, Organic Synthesis, Coll. Vol. III, 109, **1955**) to produce the intermediate compounds of formula **IV** or **V.** When it is desired to prepare compounds of formula **IV** or **V** where R³ is hydrogen, said compound may be prepared from compounds of formula **XIIa,b** via a Vilsmeier-Haack acylation, using the methods described by E. Campaigne and W. L. Archer, Organic Synthesis, Coll. Vol. IV, 331, **1963** and by J. H. Wood and R. W. Bost, Organic Synthesis, Coll. Vol. IV, 98, **1955.**

The location of the acyl group (COR³) introduced in this manner can be determined by the nature and location of the J, X and/or Y substituents present, as well as by the conditions employed for the reaction. In instances where it is desirable to prepare compounds of formula **IV** (R³ =H), from **Xlla** (E=H), introduction of the aldehyde functional group (CHO) can also be achieved using conditions described above for the preparation of the intermediates **IIa** and **IIb** in Scheme 1. For example, preparation of compounds of the formula **IV** wherein R³ = H (i.e., aldehydes) can be achieved using one or more of the known procedures for the formylation of aromatic rings, including reacting dichloromethyl methyl ether and titanium (IV) tetrachloride in methylene chloride according to the procedure described by M. L. Mancini et al., Synthetic Communications, **1989,** 2001-2007, or H. Chikashita et a!., Journal of Organic Chemistry, **1991,** 56, 1692.

For the preparation of compounds of the general formula **I** wherein R² and R³ taken together with the nitrogen to which R² is attached and the carbon to which R³ is attached form a nitrogen containing ring, an adaptation of the procedure described by L. S. Bleicher et al (Journal of Organic Chemistry, **1998**, 63, 1109) can be employed, as shown in Scheme 4. Thus, an ester of the general formula **V** (R³ = O-alkyl) (an intermediate of formula **XVIII**), prepared by esterification of the corresponding carboxylic acid of formula **V** (R³ = OH) (also of formula **XVIII**), is reacted with a cyclic lactam of the general formula **XXV** 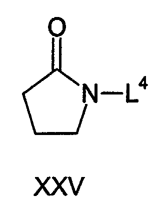 where L⁴ is a reaction labile group such as -CH=CH₂, in the presence of a strong base such as sodium methoxide, to produce the diketo intermediate of general formula **XXI**. This intermediate can then be converted to the corresponding cyclic imine of formula **XXII** in the presence of a strong acid, such as hydrochloric acid, usually under reflux conditions. Subsequently, the compounds of formula **XXII** can be reduced to form the cyclic amines of formula **XXIII** (wherein R¹ = H) using, for example, sodium borohydride in methanol as described previously. Such compounds of formula **XXIII** can further be converted into compounds of the formula **XXIII** (wherein R¹ is as defined for compounds of formula **I**) as previously discussed.

For the preparation of compounds of general formula **I,** wherein the group X is a lactam attached to phenyl or naphthyl ring A via the lactam N atom, the method illustrated in Scheme 5 is preferred. In this procedure an aldehyde or ketone of general formula **IV** (R³ = H or C₁-C₄ alkyl, respectively) where Q is NO₂ is converted to an amine of the general formula **XIX** where R¹ is as previously defined, according to the method described in Scheme 1. This intermediate **XIX** is then converted to a compound of general formula **XX,** where R² is a protecting group, preferably a *tert*-butoxy-carbonyl (*t*-BOC) group, that is stable to hydrogenation conditions but can be readily removed at a later point in the synthetic sequence; suggestions for such groups can be found in Wuts and Green, supra, at page 309. This latter intermediate **XX** wherein Q is NO₂ can then be treated under reduction conditions to form an analogous intermediate of formula **XX** wherein Q is NH₂, while leaving the *t*-BOC group intact. Such reduction conditions for this conversion are known to one skilled in the art and include the use of hydrogen gas (H₂) and a catalyst, preferably palladium on carbon, in a reaction inert solvent such as a lower alcohol (e.g., methanol, ethanol), ester (e.g., ethyl acetate), or ether (e.g., tetrahydrofuran, 1,4-dioxane) and in the presence or absence of a small amount of acid, preferably a small amount of acetic acid. The NH₂ group of the resulting compounds of formula **XX** can then be converted to cyclic amides (lactams), wherein R² remains *t*-BOC, by reacting them with an omega-chloro alkanoyl chloride or bromide or an omega-bromo alkanoyl chloride or bromide in a neutral solvent such as THF and in the presence of an acid scavenger, such as Na₂CO₃, K₂CO₃, CS₂CO₃ or the like, and heating the mixture at the boiling point of the solvent. This effects a ring closure forming the cyclic amide (i.e. lactam). Finally, the protecting group can be removed to obtain the compounds of general formula **I** wherein X is a lactam and R² is H; in the case of the *t*-BOC protecting group a mixture of ethyl acetate saturated with HCI gas is effective in such removal.

Compounds of formula XXX through XXXII and their pharmaceutically acceptable salts can be prepared as described in U.S. Patent Application No. 09/529,207, filed April 7, 2000, entitled "Monoamine Reuptake Inhibitors For Treatment of CNS Disorders," and International Patent Application No. WO 00/50380, published August 31, 2000 (of which U.S. Patent Application No. 09/529,207 is the national stage application), which claims priority from U.S. Provisional Application No. 60/121,313, filed February 23, 1999. The foregoing patent applications are incorporated herein by reference in their entirety.

This invention relates both to methods of treating alcoholism or alcohol dependence in which the monoamine reuptake inhibitor (*i.e*., the compound of formula I, XXX, XXXI or XXXII) and the opioid antagonist, or pharmaceutically acceptable salts of the same, are administered together, as part of the same pharmaceutical composition, as well as to methods in which these two active agents are administered separately as part of an appropriate dose regimen designed to obtain the benefits of the combination therapy. The appropriate dose regimen, the amount of each dose administered, and specific intervals between doses of each active agent will depend upon the subject being treated, the emetogen and the severity of the condition. Generally, in carrying out the methods of this invention, the monoamine reuptake inhibitor (*i.e*., the compound of formula I, XXX, XXXI or XXXII) will be administered to a patient in an amount ranging from about 0.01 to about 10.0 mg per kg of body weight per day. As an example, a monoamine reuptake inhibitor (*i.e*., a compound of formula I, XXX, XXXI or XXXII) will be administered to an adult human of average weight (about 70 kg) in an amount ranging from about 0.7 mg to about 700 mg per day, preferably from about 1 mg to about 500 mg per day, in single or divided doses. The compounds may be administered on a regimen of up to 6 times per day, preferably 1 to 4 times per day, especially 2 times per day and most especially once daily. A suitable dosage level for the opioid antagonist is about 0.001 to about 10.0 mg per kg of body weight per day. Thus the opioid antagonist will be administered to an adult human of average weight (about 70 kg) in an amount ranging from about 0.07 mg to about 700 mg per day, preferably from about 1 mg to about 500 mg per day, in single or divided doses. The compounds may be administered on a regimen of up to 6 time per day, preferably 1 to 4 times per day, especially 2 time per day and most especially once daily. Variations may nevertheless occur depending upon the species of animal being treated and its individual response to said medicament, as well as on the type of pharmaceutical formulation chosen and the time period and interval at which such administration is carried out. In some instances, dosage levels below the lower limit of the aforesaid range may be more than adequate, while in other cases still larger doses may be employed without causing any harmful side effect, provided that such larger doses are first divided into several small doses for administration throughout the day.

It will be appreciated that the amount of the monoamine reuptake inhibitor (*i.e*., the compound of formula I, XXX, XXXI or XXXII) and the opioid antagonist required for use in the treatment of alcoholism or alcohol dependence will vary not only with the particular compounds or compositions selected, but also with the route of administration, the nature of the condition being treated, and the age and condition of the patient, and will ultimately be at the discretion of the patient's physician or pharmacist.

When administered in combination, either as a single or as separate pharmaceutical composition(s), the monoamine reuptake inhibitor (*i.e.*, the compound of formula I, XXX, XXXI or XXXII) and the opioid antagonist, are present in a ratio which is consistent with the manifestation of the desired effect. In particular, the ratio by weight of the monoamine reuptake inhibitor (*i.e*., the compound of formula I, XXX, XXXI or XXXII) and the opioid antagonist will suitably be between 0.001 to 1 and 1000 to 1, and especially between 0.01 to I and 100 to 1.

The monoamine reuptake inhibitors (*i.e*., the compounds of formula I, XXX, XXXI and XXXII), their pharmaceutically acceptable salts, and the opioid antagonist and their pharmaceutically acceptable salts that are employed in the pharmaceutical compositions and methods of this invention are hereinafter also referred to as "therapeutic agents". The therapeutic agents can be administered via either the oral or parenteral route. Compositions containing both a monoamine reuptake inhibitor (*i.e*., a compound of formula I, XXX, XXXI or XXXII) and an opiod antagonist, or pharmaceutically acceptable salts of one or both therapeutic agents, will generally be administered orally or parenterally daily, in single or divided doses, so that the total amount of each active agent administered falls within the above guidelines.

The therapeutic agents may be administered alone or in combination with pharmaceutically acceptable carriers or diluents by either of the routes previously indicated, and such administration may be carried out in single or multiple doses. More particularly, the therapeutic agents of this invention can be administered in a wide variety of different dosage forms, *i.e*., they may be combined with various pharmaceutically acceptable inert carriers in the form of tablets, capsules, lozenges, troches, hard candies, suppositories, aqueous suspensions, injectable solutions, elixirs, syrups, and the like. Such carriers include solid diluents or fillers, sterile aqueous media and various non-toxic organic solvents, etc. Moreover, oral pharmaceutical compositions can be suitably sweetened and/or flavored. In general, the therapeutic agents of this invention, when administered separately (*i.e*., not in the same pharmaceutical composition) are present in such dosage forms at concentration levels ranging from about 5.0% to about 70% by weight.

For oral administration, tablets containing various excipients such as microcrystalline cellulose, sodium citrate, calcium carbonate, dicalcium phosphate and glycine may be employed along with various disintegrants such as starch (and preferably corn, potato or tapioca starch), alginic acid and certain complex silicates, together with granulation binders like polyvinylpyrrolidone, sucrose, gelatin and acacia. Additionally, lubricating agents such as magnesium stearate, sodium lauryl sulfate and talc are often very useful for tabletting purposes. Solid compositions of a similar type may also be employed as fillers in gelatin capsules; preferred materials in this connection also include lactose or milk sugar as well as high molecular weight polyethylene glycols. When aqueous suspensions and/or elixirs are desired for oral administration, the active ingredient may be combined with various sweetening or flavoring agents, coloring matter or dyes, and, if so desired, emulsifying and/or suspending agents as well, together with such diluents as water, ethanol, propylene glycol, glycerin and various like combinations thereof.

For parenteral administration, solutions of a therapeutic agent in either sesame or peanut oil or in aqueous propylene glycol may be employed. The aqueous solutions should be suitably buffered if necessary and the liquid diluent first rendered isotonic. These aqueous solutions are suitable for intravenous injection purposes. The oily solutions are suitable for intraarticular, intramuscular and subcutaneous injection purposes. The preparation of all these solutions under sterile conditions is readily accomplished by standard pharmaceutical techniques well known to those skilled in the art.

As stated above, the monoamine reuptake inhibitor (*i.e*., the compound of formula I, XXX, XXXI or XXXII) and the opioid antagonist may be formulated in a single pharmaceutical composition or alternatively in individual pharmaceutical compositions for simultaneous, separate or sequential use in accordance with the present Invention.

Preferably the compositions according to the present invention, which contain both a monoamine reuptake inhibitor (*i.e*., a compound of formula I, XXX, XXXI or XXXII) and an opioid antagonist, as well as the pharmaceutical compositions used to deliver only one of these active agents, are in unit dosage forms such as tablets, pills, capsules, powders, granules, solutions or suspensions, or suppositories, for oral, parenteral or rectal administration, by inhalation or insufflation or administration by transdermal patches or by buccal cavity absorption wafers.

For preparing solid compositions such as tablets, the principal active ingredient is mixed with a pharmaceutical carrier, *e.g.,* conventional tableting ingredients such as corn starch, lactose, sucrose, sorbitol, talc, stearic acid, magnesium stearate, dicalcium phosphate or gums, and other pharmaceutical diluents, *e.g.*, water, to form a solid preformulation composition containing a homogeneous mixture of a compound of the present invention, or a non-toxic pharmaceutically acceptable salt thereof. When referring to these preformulation compositions as homogeneous, it is meant that the active ingredient is dispersed evenly throughout the composition so that the composition may be readily subdivided into equally effective unit dosage forms such as tablets, pills and capsules. This solid preformulation composition is then subdivided into unit dosage forms of the type described above containing, typically, from about 0.05 mg to about 500 mg of each of the therapeutic agents contained in the composition. The tablets or pills of the composition can be coated or otherwise compounded to provide a dosage form affording the advantage of prolonged action. For example, the tablet or pill can comprise an inner dosage and an outer dosage component, the latter being in the form of an envelope over the former. The two components can be separated by an enteric layer that serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac acetyl alcohol and cellulose acetate.

The liquid forms in which the novel compositions of the present invention may be incorporated for administration orally or by injection include aqueous solutions, suitably flavored syrups, aqueous or oil suspensions, and flavored emulsions with edible oils such as cottonseed oil, sesame oil, coconut oil, peanut oil or soybean oil, as well as elixirs and similar pharmaceutical vehicles. Suitable dispersing or suspending agents for aqueous suspensions include synthetic and natural gums such as tragacanth, acacia, alginate, dextran, sodium carboxymethyl cellulose, methylcellulose, polyvinyl-pyrrolidone or gelatin.

Preferred compositions for administration of a monoamine reuptake inhibitor (*i.e*., a compound of formula I, XXX, XXXI or XXXII), or other therapeutic agent by injection include those comprising the therapeutic agent in association with a surface-active agent (or wetting agent or surfactant) or in the form of an emulsion (as a water-in-oil or oil-in-water emulsion).

Suitable surface-active agents include, in particular, non-ionic agents, such as polyoxyethylenesorbitans (*e.g.*, Tween™ 20, 40, 60, 80 or 85) and other sorbitans (*e.g.*, Span™ 20, 40, 60, 80 or 85). Compositions with a surface-active agent will conveniently comprise between 0.05 and 5% surface-active agent, and preferably between 0.1 and 2.5%. It will be appreciated that other ingredients may be added, for example mannitol or other pharmaceutically acceptable vehicles, if necessary.

Suitable emulsions may be prepared using commercially available fat emulsions, such as Intralipid™, Liposyn™, Infonutrol™, Lipofundin ™ and Lipiphysan™. The therapeutic agent may be either dissolved in a pre-mixed emulsion composition or, alternatively, it may be dissolved in an oil (*e.g.*, soybean oil, safflower oil, cottonseed oil, sesame oil, com oil or almond oil) and an emulsion formed upon mixing with a phospholipid (*e.g.*, eggs phospholipids, soybean phospholipids or soybean lecithin) and water. It will be appreciated that other ingredients may be added, for example glycerol or glucose, to adjust the tonicity of the emulsion. Suitable emulsions will typically contain up to 20% oil, for example, between 5 and 20%. The fat emulsion will preferably comprise fat droplets between 0.1 and 1.0 µm, particularly 0.1 and 0.5 µm, and have a pH in the range of 5.5 to 8.0.

Compositions for inhalation or insufflation include solutions and suspensions in pharmaceutically acceptable aqueous or organic solvents, or mixtures thereof, and powders. The liquid or solid compositions may contain suitable pharmaceutically acceptable excipients as set out above. Preferably the compositions are administered by the oral or nasal respiratory route for local or systemic effect. Compositions in preferably sterile pharmaceutically acceptable solvents may be nebulised by use of inert gases. Nebulised solutions may be breathed directly from the nebulising device or the nebulising devise may be attached to a facemask, tent or intermittent positive pressure breathing machine. Solution, suspension, or powder compositions may be administered, preferably orally or nasally, from devices that deliver the formulation in an appropriate manner.

Compositions of the present invention may also be presented for administration in the form of transdermal patches using conventional technology. The compositions may also be administered via the buccal cavity using, for example, absorption wafers.

The present invention further provides a process for the preparation of a pharmaceutical composition comprising a monoamine reuptake inhibitor (*i.e*., a compound of formula I, XXX, XXXI or XXXII) and an opioid antagonist, or pharmaceutically acceptable salts of the same, which process comprises bringing a monoamine reuptake inhibitor (*i.e*., a compound of formula I, XXX, XXXI or XXXII) and the opioid antagonist (or the pharmaceutically acceptable salts of one or both of these therapeutic agents) into association with a pharmaceutically acceptable carrier or excipient.

The compounds of formula I and XXX through XXXII, and their pharmaceutically acceptable salts, are useful as monoamine reuptake inhibitors, *i.e*., they possess the ability to inhibit the reuptake of serotonin, dopamine and norepinephrine at the individual monoamine reuptake sites in mammals, and therefore, they are able to function as therapeutic agents in the treatment of the aforementioned disorders and diseases in an afflicted mammal.

The ability of compounds of formula I and XXX through XXXII to inhibit the reuptake of serotonin, dopamine and norepinephrine at the individual monoamine reuptake sites may be determined using the following procedures. The *in vitro* activity of the compounds of formula I and XXX through XXXII at the individual monoamine reuptake sites can be determined using rat synaptosomes or HEK-293 cells transfected with the human serotonin, dopamine or norepinephrine transporter, according to the following procedure adapted from those described by S. Snyder *et al.,* (Molecular Pharmacology, 1971, 7, 66-80), D.T. Wong *et al*., (Biochemical Pharmacology, 1973, 22, 311-322), H. F. Bradford (Journal of Neurochemistry, 1969, 16, 675-684) and D. J. K. Balfour (European Journal of Pharmacology, 1973, 23, 19-26).

Synaptosomes: Male Sprague Dawley rats are decapitated and the brains rapidly removed. The cortex, hippocampi and corpus striata are dissected out and placed in ice cold sucrose buffer, 1 gram in 20 ml of buffer (the buffer is prepared using 320 mM sucrose containing 1mg/ml glucose, 0.1mM ethylenediamine tetraacetic acid (EDTA) adjusted to pH 7.4 with tris(hydroxymethyl)-aminomethane (TRIS) base). The tissues are homogenized in a glass homogenizing tube with a Teflon™ pestle at 350 rpm using a Potters homogenizer. The homogenate is centrifuged at 1000 x g for 10 min. at 4°C. The resulting supernatant is recentrifuged at 17,000 x g for 20 min. at 4°C. The final pellet is resuspended in an appropriate volume of sucrose buffer that yielded less than 10% uptake.

Cell Preparation: HEK-293 cells transfected with the human serotonin (5-HT), norepinephrine (NE) or dopamine (DA) transporter are grown in DMEM (Dulbecco's Modified Eagle Medium, Life Technologies Inc., 9800 Medical Center Dr., Gaithersburg, MD, catalog no. 11995-065)) supplemented with 10% dialyzed FBS (Fetal Bovine Serum, from Life Technologies, catalog no. 26300-053), 2 mM L-glutamine and 250 ug/ml G418 for the 5-HT and NE transporter or 2ug/ml puromycin for the DA transporter, for selection pressure. The cells are grown in Gibco triple flasks, harvested with Phosphate Buffered Saline (Life Technologies, catalog no. 14190-136) and diluted to an appropriate amount to yield less than 10% uptake.

Neurotransmitter Uptake Assay: The uptake assays are conducted in glass tubes containing 50 uL of solvent, inhibitor or 10uM sertraline, desipramine or nomifensine for the 5-HT, NE or DA assay nonspecific uptake, respectively. Each tube contains 400 uL of [3H]5-HT (5 nM final), [3H]NE (10 nM final) or [3H]DA (5 nM final) made up in modified Krebs solution containing 100 uM pargyline and glucose (1mg/ml). The tubes are placed on ice and 50 uL of synaptosomes or cells is added to each tube. The tubes are then incubated at 37° C for 7 min. (5-HT, DA) or 10 min. (NE). The incubation is terminated by filtration (GF/B filters), using a 96-well Brandel Cell Harvester, the filters are washed with modified Krebs buffer and counted using either a Wallac Model 1214 or Wallac Beta Plate Model 1205 scintillation counter.

Determination of the *in vivo* serotonin reuptake inhibition activity and potency of action for the compounds used in the methods and pharmaceutical compositions of the present invention can be made by measuring the ability of the compound to block the depletion of serotonin in the anterior cortex induced by (+/-)-para-chloroamphetamine (PCA) in the rat, according to a procedure adapted from R. W. Fuller, H. D. Snoddy and M. L. Cohen in Neuropharmacology 23: 539-544 (1984).

Generally, male, white Sprague-Dawley rats weighing 160-230 g each are assigned to either the control (vehicle) or test groups. When the test compound is administered subcutaneously (sc) at a given dose, it is co-administered with 5 mg/kg of parachloroamphetamine (PCA). Three hours post-dose, the animals are sacrificed by decapitation and the anterior cortices are removed, wrapped in parafilm and frozen in dry ice (-78 C). When dosed orally (po), the rats are fasted the night before the experiment and then treated with the test compound at a given dose 30 minutes prior to the administration of the PCA (5 mg/kg, sc). After three hours, the animals are sacrificed and the tissues removed as above.

To determine the serotonin (5-HT) levels, the frozen tissues are homogenized with Branson sonifier in 0.5 mL of mobile phase in Eppendorf centrifuge tubes. Samples are then spun down at 11000 rpm for twenty minutes in a Sorval SH-MT rotor in a Sorval RC5C centrifuge. The supernatant thus obtained is pipetted into HPLC vials and the 5-HT levels are measured on HPLC-EC.

Interpretation of the results is as follows: Each experiment has a set of vehicle treated animals and a set of PCA-only animals. The mean 5-HT value of the PCA animals is subtracted from the mean 5-HT value of the vehicle animals. This is the signal or window of the response. The mean 5-HT value of each test group is determined, the mean of the PCA group subtracted from that, and that amount divided by the window is the per cent protection from the PCA effect for that dose. To report an ID₅₀, a line is drawn mathematically through the per cent protection values and the 50 per cent level calculated.

Compounds which are opioid antagonists, including the examples of specific opioid antagonists referred to herein, may be used in the treatment of disorders and conditions that can be treated by modulating binding to the opioid receptor. Thus, compounds which are opioid antagonists, including the examples of specific opioid antagonists referred to herein, are able to function as therapeutic agents in the treatment of the aforementioned disorders and diseases in an afflicted mammal.

The ability of opioid antagonists to bind to the various opioid receptors and their functional activity at such receptors can be determined as described below. Binding to the delta opioid receptor can be determined using procedures well known in the art, such as those referred to by Lei Fang et al., J. Pharm. Exp. Ther., 268, 1994, 836 - 846 and Contreras et al., Brain Research, 604, 1993, 160 - 164.

In the description of binding and functional assays that follows, the following abbreviations and terminology are used.

DAMGO is [D-Ala2,N-MePhe4,Gly5-ol]enkephalin).

U69593 is ((5a, 7a, 8b)-(+)-N-methyl-N-(7-[1-pyrrolidinyl]-1-oxasipro[4,5]dec-8-yl)-benzeneacetamide).

SNC-80 is (+)-4-[(αR)-α((2S,5R)-4-allyl-2,5-dimethyl-1-piperazinyl)-3-methoxybenzyl]-N,N-diethylbenzamide.

nor BNI is nor-binaltorphimine.

CTOP is 1,2-Dithia-5,8,11,14,17-pentaazacycloeicosane, cyclic peptide derivative DPDPE is [D-en2,D-Pen5]enkephalin).

[3H]-DAMGO, [3H]-U69593, norBNI, and CTOP are all commercially available from DuPont, Amersham International, RBI and DuPont, Amersham International, RBI and DuPont respectively.

[3H]-SNC80 was prepared by Amersham International.

Opioid (mu and kappa) receptor binding assays can be performed in guinea-pig brain membrane preparations. Binding assays can be carried out at 25°C for 60 minutes in 50 mM Tris (pH 7.4) buffer. [³H]-DAMGO(2 nM) and [³H]-U-69,593 (2 nM) can be used to label mu and kappa receptor binding sites, respectively. The protein concentration can be approximately 200 µg/well. Non-specific binding can be defined with 10 µM naloxone.

Delta receptor binding assays can be performed in a stable line of CHO cells expressing the human delta receptor. The binding assay can be carried out at 25°C for 120 minutes in 50 mM Tris (pH 7.4) buffer. [³H]-SNC-80 can be used to label delta receptor binding sites. The protein concentration can be approximately 12.5 µg/well. Non-specific binding can be defined with 10 µM naltrexone.

The binding reaction can be terminated by rapid filtration through glass fiber filters, and the samples can be washed with ice-cold 50 mM Tris buffer (pH 7.4).

Agonist activity at the delta, mu and kappa opioid receptors can be determined as follows.

Opioid (delta, mu and kappa) activity is studied, as described below, in two isolated tissues, the mouse deferens (MVD)(δ) and the guinea-pig myentric plexus with attached longitudinal muscle (GPMP) (µ and k).

MVD (DC1 strain, Charles River, 25-35 g) are suspended in 15 ml organ baths containing Mg⁺⁺ free Krebs' buffer of the following composition (mM): NaCl, 119; KCI, 4.7; NaHCO₃, 25; KH₂PO₄, 1.2; CaCl₂, 2,5 and glucose, 11. The buffer is gassed with 95% O₂ and 5% CO₂. The tissues are suspended between platinum electrodes, attached to an isometric transducer with 500 mg tension, and stimulated with 0.03 Hz pulses of 1-msec pulse-width at supramaximal voltage. IC₅₀ values are determined by the regression analysis of concentration-response curves for inhibition of electrically-induced contractions in the presence of 300 nM of the mu-selective antagonist CTOP. This test is a measure of δ agonism.

Guinea-pig (Porcellus strain, male, 450-500 g, Dunkin Hartley) myentric plexus with attached longitudinal muscle segments are suspended with 1 g of tension in Krebs' buffer and stimulated with 0.1 Hz pulses of 1-msec pulse-width at supramaximal voltage. Mu functional activity is determined in the presence of 10 nM nor-BNI with 1 µM of the mu selective agonist, DAMGO, added to the bath at the end of the experiment to define a maximal response. This test is a measure of mu agonism.

Kappa functional activity is determined in the presence of and 1 µM CTOP with 1 µM of the kappa selective agonist U-69,593 added at the end of the experiment to define a maximal response. All inhibitions of twitch height for test compounds are expressed as a percentage of the inhibition obtained with the standard agonist and the corresponding IC₅₀ values determined.

The following procedure can be used to determine the activity of the compounds employed in the methods and pharmaceutical compositions of this invention as antagonists and/or as agonists of delta opioid receptors.

Cell Culture: Chinese hamster ovary cells expressing the human delta opioid receptor are passaged twice weekly in Hamis F-12 media with L-glutamine containing 10% fetal bovine serum and 450 µg/mL hygromycin. Cells are prepared for assays 3 days prior to the experiment. 15 mL of 0.05% trypsin/EDTA is added to a confluent triple flask, swirled and decanted to rinse. 15 mL of 0.05% trypsin/EDTA is again added, and the flask is placed into a 37C incubator for 2 minutes. Cells are removed from the flask by banking, and the supernatant is poured off into a 50 mL tube. 30 mL of media is then added to the flask to stop the action of the trypsin, and then decanted into the 50 mL tube. The tube is then centrifuged for 5 minutes at 1000 rpm, the media is decanted, and the pellet is resuspended in 10 mL of media. Viability of the cells is assessed using trypan blue, and the cells are counted and plated out into 96 well poly-D-lysine coated plates at a density of 7,500 cells/well.

Antagonist Test Plate: Cells plated 3 days prior to assay are rinsed twice with PBS. The plates are placed into a 37°C water bath. 50 µL of assay buffer (PBS, dextrose 1 mg/mL, 5mM MgCl₂, 30 mM HEPES, 66.7 µg/mL of IBMX) is then added to designated wells. Fifty microliters of appropriate drug is then added to designated wells and timed for 1 minute. Fifty microliters of 10 µM forskolin + 0.4nM DPDPE (final assay concentration is 5 µM forskolin, 0.2nM DPDPE) is then added to appropriate wells and timed for 15 minutes. The reaction is stopped by the addition of 10 µL of 6N perchloric acid to all wells. To neutralize, 13 µL of 5N KOH is added to all wells, and to stabilize, 12 µL of 2M Tris, pH 7.4, is added to all wells. The contents of the wells are mixed by shaking on an orbital shaker for 10 minutes and centrifuged at setting 7 for 10 minutes. Aliquot into 3H plate.

Agonist Test Plate: Cells plated 3 days prior to assay are rinsed twice with PBS. The plates are placed into a 37°C water bath. Fifty microliters of assay buffer (PBS, dextrose 1 mg/mL, 5mM MgCl₂, 30mM HEPES, 66.7 µg/mL of IBMX) is then added to designated wells. Fifty microliters of appropriate drug + 10 µM forskolin (final assay concentration is 5µM forskolin) is then added to all wells and timed for 15 minutes. The reaction is then stopped by the addition of 10 µL of 6N perchloric acid to all wells. To neutralize, 13 µ of 5N KOH is added to all wells, and to stabilize, 12 µL of 2M Tris, pH 7.4, is added to all wells. The contents of the wells are mixed by shaking on an orbital shaker for 10 minutes and centrifuged at setting 7 for 10 minutes. Aliquot into 3H plate.

Both test plates are placed into an Amersham 3H cAMP binding kit overnight. The cells are harvested onto GF/B filters previously soaked in 0.5% PEI with a Skatron, using 50 mM Tris HCI, pH 7.4, at 4°C. Filtermats can be air-dried overnight then placed in bags with 20 ml Betaplate scintillation cocktail and counted on a Betaplate counter for 60 sec per sample.

## Claims

1. A pharmaceutical composition for the treatment of alcoholism or alcohol dependence in a mammal, comprising: (a) an opioid antagonist, or a pharmaceutically acceptable salt thereof; (b) a compound of the formula I, as depicted and defined below, 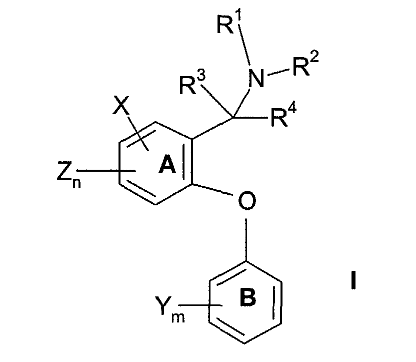 or pharmaceutically acceptable salt thereof, wherein phenyl ring A and phenyl ring B can each, independently, be replaced by a naphthyl group, and wherein when phenyl ring A is replaced by a naphthyl group, the ethereal oxygen of structure I and the carbon to which R³, R⁴ and NR¹R² are attached, are attached to adjacent ring carbon atoms of the naphthyl group and neither of said adjacent ring carbon atoms is also adjacent to a fused ring carbon atom of said naphthyl group;
n and m are selected, independently, from one, two and three;
R¹ and R² are selected, independently, from hydrogen, (C₁-C₄)alkyl, (C₂-C₄)alkenyl, and (C₂-C₄)alkynyl, or R¹ and R², together with the nitrogen to which they are attached, form a four to eight membered saturated ring containing one or two heteroatoms, including the nitrogen to which R¹ and R² are attached, wherein the second heteroatom, when present, is selected from oxygen, nitrogen and sulfur, and wherein said ring may optionally be substituted at available binding sites with from one to three substituents selected, independently, from hydroxy and (C₁-C₆)alkyl;
R³ and R⁴ are selected, independently, from hydrogen and (C₁-C₄) alkyl optionally substituted with from one to three fluorine atoms, or R³ and R⁴ together with the carbon to which they are attached form a four to eight membered saturated carbocyclic ring, and wherein said ring may optionally be substituted at available binding sites with from one to three substituents selected, independently, from hydroxy and (C₁-C₆)alkyl;
or R² and R³, together with the nitrogen to which R² is attached and the carbon to which R³ is attached, form a four to eight membered saturated ring containing one or two heteroatoms, including the nitrogen to which R² is attached, wherein the second heteroatom, when present, is selected from oxygen, nitrogen and sulfur, and wherein said ring may optionally be substituted at available binding sites with from one to three substituents selected, independently, from hydroxy and (C₁-C₆)alkyl;
each X is selected, independently, from phenyl, heteroaryl and heterocyclic groups, and may be further substituted by hydrogen, halo, (C₁-C₄)alkyl optionally substituted with from one to three fluorine atoms, (C₁-C₄)alkoxy optionally substituted with from one to three fluorine atoms, cyano, nitro, amino, hydroxy, carbonyl, (C₁-C₄)alkylamino, di-[(C₁-C₄)alkyl]amino, NR⁵(C=O)(C₁-C₄)alkyl, SO₂NR⁵R⁶ or SOₚ(C₁-C₆)alkyl, wherein R⁵ and R⁶ are selected, independently, from hydrogen and (C₁-C₆)alkyl, and p is zero, one or two;
each Y is selected, independently, from hydrogen, halo, (C₁-C₄)alkyl optionally substituted with from one to three fluorine atoms, (C₁-C₄)alkoxy optionally substituted with from one to three fluorine atoms, cyano, nitro, amino, (C₁-C₄)alkylamino, di-[(C₁-C₄)alkyl]amino, NR⁵(C=O)(C₁-C₄)alkyl, SO₂NR⁵R⁶ and SOₚ(C₁-C₆)alkyl, wherein R⁵ and R⁶ are selected, independently, from hydrogen and (C₁-C₆)alkyl, and p is zero, one or two; and
each Z is selected independently from hydrogen, halo, (C₁-C₄)alkyl optionally substituted with from one to three fluorine atoms, and (C₁-C₄)alkoxy;
or a compound of the formula XXX, as depicted and defined below, 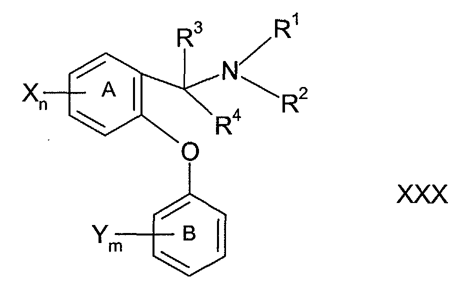 or pharmaceutically acceptable salt thereof, wherein phenyl ring A and phenyl ring B can each, independently, be replaced by a naphthyl group, and wherein when phenyl ring A is replaced by a naphthyl group, the ethereal oxygen of structure I and the carbon to which R³, R⁴ and NR¹R² are attached, are attached to adjacent ring carbon atoms of the naphthyl group and neither of said adjacent ring carbon atoms is also adjacent to a fused ring carbon atom of said naphthyl group;
n and m are, selected, independently, from one, two and three;
R¹ and R² are selected, independently, from hydrogen, (C₁-C₄)alkyl, (C₂-C₄)alkenyl, and (C₂-C₄)alkynyl, or R¹ and R², together with the nitrogen to which they are attached, form a four to eight membered saturated ring containing one or two heteroatoms, including the nitrogen to which R¹ and R² are attached, wherein the second heteroatom, when present, is selected from oxygen, nitrogen and sulfur, with the proviso that said ring can not contain two adjacent oxygen atoms or two adjacent sulfur atoms, and wherein said ring may optionally be substituted at available binding sites with from one to three substituents selected, independently, from hydroxy and (C₁-C₆)alkyl;
R³ and R⁴ are selected, independently, from hydrogen and (C₁-C₄) alkyl optionally substituted with from one to three fluorine atoms, or R³ and R⁴, together with the carbon to which they are attached, form a four to eight membered saturated carbocyclic ring, and wherein said ring may optionally be substituted at available binding sites with from one to three substituents selected, independently, from hydroxy and (C₁-C₆)alkyl;
or R² and R³, together with the nitrogen to which R² is attached and the carbon to which R³ is attached, form a four to eight membered saturated ring containing one or two heteroatoms, including the nitrogen to which R² is attached, wherein the second heteroatom, when present, is selected from oxygen, nitrogen and sulfur, with the proviso that said ring can not contain two adjacent oxygen atoms or two adjacent sulfur atoms, and wherein said ring may optionally be substituted at available binding sites with from one to three substituents selected, independently, from hydroxy and (C₁-C₆)alkyl;
each X is selected, independently, from hydrogen, halo, (C₁-C₄)alkyl optionally substituted with from one to three fluorine atoms, (C₁-C₄)alkoxy optionally substituted with from one to three fluorine atoms, cyano, nitro, amino, (C₁-C₄)alkylamino, di-[(C₁-C₄)alkyl]amino, NR⁵(C=O)(C₁-C₄)alkyl, SO₂NR⁵R⁶ and SOₚ(C₁-C₆)alkyl, wherein R⁵ and R⁶ are selected, independently, from hydrogen and (C₁-C₆)alkyl, and p is zero, one or two; and
each Y is selected, independently, from hydrogen, (C₁-C₆)alkyl and halo;
with the proviso that: (1) no more than one of NR¹R², CR³R⁴ and R²NCR³ can form a ring; and (2) at least one X must be other than hydrogen when (i) R³ and R⁴ are both hydrogen, (ii) R¹ and R² are selected, independently, from hydrogen and (C₁-C₄)alkyl, and (iii) ring B is mono- or disubstituted with, respectively, one or two halo groups;
or a compound of the formula XXXI or XXXII, as depicted below, 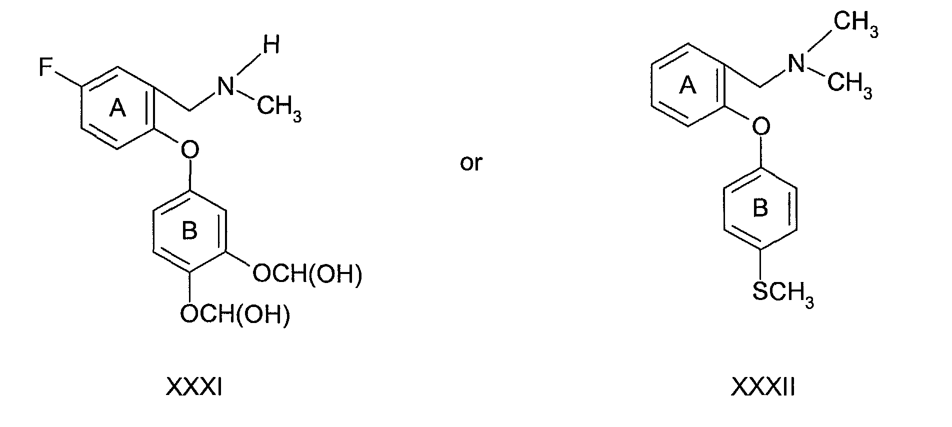 or pharmaceutically acceptable salt thereof; and (c) a pharmaceutically acceptable carrier; wherein the active agents "a" and "b" above are present in amounts that render the composition effective in treating alcoholism or alcohol dependence.

2. A pharmaceutical composition according to claim 1, wherein the amount of the opioid antagonist, or pharmaceutically acceptable salt thereof, in said composition is from about 0.07 mg to about 700 mg and the amount of the compound of formula I or formula XXX or formula XXXI or XXXII, respectively, or pharmaceutically acceptable salt thereof, is from about 0.7 mg to about 700 mg.

3. A pharmaceutical composition according to claim 2, wherein the amount of the opioid antagonist, or pharmaceutically acceptable salt thereof, in said composition is from about 1 mg to about 500 mg and the amount of the compound of formula I or formula XXX or formula XXXI or XXXII, respectively, or pharmaceutically acceptable salt thereof, is from about 1 mg to about 500 mg.

4. Use of a pharmaceutical composition according to any of claims 1 to 3 for the manufacture of a medicament for the treatment of alcoholism or alcohol dependence in a mammal.

5. Use of a combination of: (a) an opioid antagonist, or a pharmaceutically acceptable salt thereof; and (b) a compound of the formula I, as depicted and defined below, 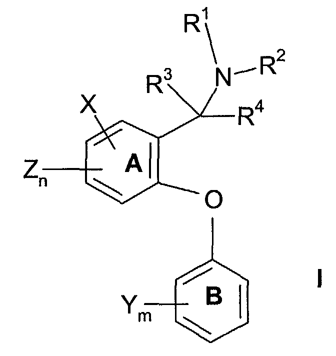 or pharmaceutically acceptable salt thereof, for the manufacture of a medicament for the treatment of alcoholism or alcohol dependence in a mammal, wherein phenyl ring A and phenyl ring B can each, independently, be replaced by a naphthyl group, and wherein when phenyl ring A is replaced by a naphthyl group, the ethereal oxygen of structure I and the carbon to which R³, R⁴ and NR¹R² are attached, are attached to adjacent ring carbon atoms of the naphthyl group and neither of said adjacent ring carbon atoms is also adjacent to a fused ring carbon atom of said naphthyl group;
n and m are selected, independently, from one, two and three;
R¹ and R² are selected, independently, from hydrogen, (C₁-C₄)alkyl, (C₂-C₄)alkenyl, and (C₂-C₄)alkynyl, or R¹ and R², together with the nitrogen to which they are attached, form a four to eight membered saturated ring containing one or two heteroatoms, including the nitrogen to which R¹ and R² are attached, wherein the second heteroatom, when present, is selected from oxygen, nitrogen and sulfur, and wherein said ring may optionally be substituted at available binding sites with from one to three substituents selected, independently, from hydroxy and (C₁-C₆)alkyl;
R³ and R⁴ are selected, independently, from hydrogen and (C₁-C₄) alkyl optionally substituted with from one to three fluorine atoms, or R³ and R⁴ together with the carbon to which they are attached form a four to eight membered saturated carbocyclic ring, and wherein said ring may optionally be substituted at available binding sites with from one to three substituents selected, independently, from hydroxy and (C₁-C₆)alkyl;
or R² and R³, together with the nitrogen to which R² is attached and the carbon to which R³ is attached, form a four to eight membered saturated ring containing one or two heteroatoms, including the nitrogen to which R² is attached, wherein the second heteroatom, when present, is selected from oxygen, nitrogen and sulfur, and wherein said ring may optionally be substituted at available binding sites with from one to three substituents selected, independently, from hydroxy and (C₁-C₆)alkyl;
each X is selected, independently, from phenyl, heteroaryl and heterocyclic groups, and may be further substituted by hydrogen, halo, (C₁-C₄)alkyl optionally substituted with from one to three fluorine atoms, (C₁-C₄)alkoxy optionally substituted with from one to three fluorine atoms, cyano, nitro, amino, hydroxy, carbonyl, (C₁-C₄)alkylamino, di-[(C₁-C₄)alkyl]amino, NR⁵(C=O)(C₁-C₄)alkyl, SO₂NR⁵R⁶ or SOₚ(C₁-C₆)alkyl, wherein R⁵ and R⁶ are selected, independently, from hydrogen and (C₁-C₆)alkyl, and p is zero, one or two;
each Y is selected, independently, from hydrogen, halo, (C₁-C₄)alkyl optionally substituted with from one to three fluorine atoms, (C₁-C₄)alkoxy optionally substituted with from one to three fluorine atoms, cyano, nitro, amino, (C₁-C₄)alkylamino, di-[(C₁-C₄)alkyl]amino, NR⁵(C=O)(C₁-C₄)alkyl, SO₂NR⁵R⁶ and SOₚ(C₁-C₆)alkyl, wherein R⁵ and R⁶ are selected, independently, from hydrogen and (C₁-C₆)alkyl, and p is zero, one or two; and
each Z is selected independently from hydrogen, halo, (C₁-C₄)alkyl optionally substituted with from one to three fluorine atoms, and (C₁-C₄)alkoxy;
or a compound of the formula XXX, as depicted and defined below, 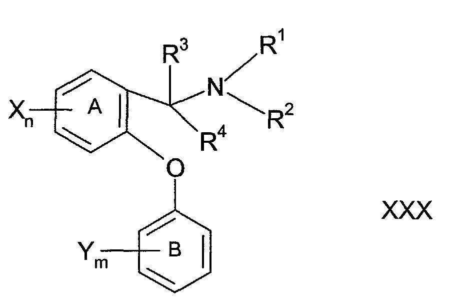 or pharmaceutically acceptable salt thereof, wherein phenyl ring A and phenyl ring B can each, independently, be replaced by a naphthyl group, and wherein when phenyl ring A is replaced by a naphthyl group, the ethereal oxygen of structure I and the carbon to which R³, R⁴ and NR¹R² are attached, are attached to adjacent ring carbon atoms of the naphthyl group and neither of said adjacent ring carbon atoms is also adjacent to a fused ring carbon atom of said naphthyl group;
n and m are, selected, independently, from one, two and three;
R¹ and R² are selected, independently, from hydrogen, (C₁-C₄)alkyl, (C₂-C₄)alkenyl, and (C₂-C₄)alkynyl, or R¹ and R², together with the nitrogen to which they are attached, form a four to eight membered saturated ring containing one or two heteroatoms, including the nitrogen to which R¹ and R² are attached, wherein the second heteroatom, when present, is selected from oxygen, nitrogen and sulfur, with the proviso that said ring can not contain two adjacent oxygen atoms or two adjacent sulfur atoms, and wherein said ring may optionally be substituted at available binding sites with from one to three substituents selected, independently, from hydroxy and (C₁-C₆)alkyl;
R³ and R⁴ are selected, independently, from hydrogen and (C₁-C₄) alkyl optionally substituted with from one to three fluorine atoms, or R³ and R⁴, together with the carbon to which they are attached, form a four to eight membered saturated carbocyclic ring, and wherein said ring may optionally be substituted at available binding sites with from one to three substituents selected, independently, from hydroxy and (C₁-C₆)alkyl;
or R² and R³, together with the nitrogen to which R² is attached and the carbon to which R³ is attached, form a four to eight membered saturated ring containing one or two heteroatoms, including the nitrogen to which R² is attached, wherein the second heteroatom, when present, is selected from oxygen, nitrogen and sulfur, with the proviso that said ring can not contain two adjacent oxygen atoms or two adjacent sulfur atoms, and wherein said ring may optionally be substituted at available binding sites with from one to three substituents selected, independently, from hydroxy and (C₁-C₆)alkyl;
each X is selected, independently, from hydrogen, halo, (C₁-C₄)alkyl optionally substituted with from one to three fluorine atoms, (C₁-C₄)alkoxy optionally substituted with from one to three fluorine atoms, cyano, nitro, amino, (C₁-C₄)alkylamino, di-[(C₁-C₄)alkyl]amino, NR⁵(C=O)(C₁-C₄)alkyl, SO₂NR⁵R⁶ and SOₚ(C₁-C₆)alkyl, wherein R⁵ and R⁶ are selected, independently, from hydrogen and (C₁-C₆)alkyl, and p is zero, one or two; and
each Y is selected, independently, from hydrogen, (C₁-C₆)alkyl and halo;
with the proviso that: (1) no more than one of NR¹R², CR³R⁴ and R²NCR³ can form a ring; and (2) at least one X must be other than hydrogen when (i) R³ and R⁴ are both hydrogen, (ii) R¹ and R² are selected, independently, from hydrogen and (C₁-C₄)alkyl, and (iii) ring B is mono- or disubstituted with, respectively, one or two halo groups;
or a compound of the formula XXXI or XXXII, respectively, as depicted below, 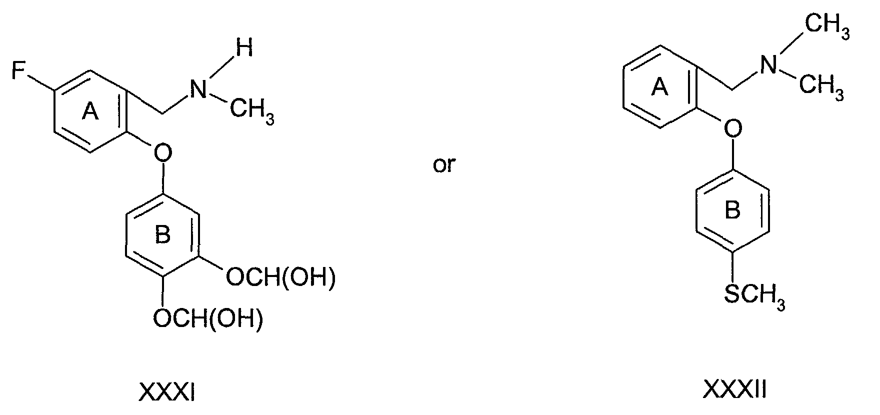 or pharmaceutically acceptable salt thereof; wherein the active agents "a" and "b" above are present in amounts that render the combination of the two agents effective in treating alcoholism or alcohol dependence.

6. A use according to claim 5, wherein the opioid antagonist, or pharmaceutically acceptable salt thereof, and the compound of formula I or formula XXX or formula XXXI or XXXII, respectively, or pharmaceutically acceptable salt thereof, are combined as part of the same dosage form.

7. A use according to claim 5 or claim 6, wherein the compound of formula I or formula XXX or formula XXXI or XXXII, respectively, or pharmaceutically acceptable salt thereof, is in an amount ranging from about 0.7 mg per day to about 700 mg per day, and the opioid antagonist, or pharmaceutically acceptable salt thereof, is in an amount ranging from about 0.07 mg per day to about 700 mg per day.

8. A use according to claim 7, wherein the compound of formula I or formula XXX or formula XXXI or XXXII, respectively, or pharmaceutically acceptable salt thereof, is in an amount ranging from about 1 mg per day to about 500 mg per day.

9. A pharmaceutical composition according to claim 1, wherein the compound of formula I or formula XXX, respectively, or pharmaceutically acceptable salt thereof, that is employed in such composition is selected from the following compounds and their pharmaceutically acceptable salts:
[4-(3,4-Dichlorophenoxy)-biphenyl-3-ylmethyl]-methylamine;
[2-(3,4-Dichlorophenoxy)-5-thiophen-3-ylbenzyl]-methylamine;
[2-(3,4-Dichlorophenoxy)-4-thiophen-3-ylbenzyl]-methylamine;
[2-(3,4-Dichlorophenoxy)-4-furan-2-ylbenzyl]-methylamine;
[2-(3,4-Dichlorophenoxy)-5-furan-2-ylbenzyl]-methylamine;
N-[4'-(3,4-Dichlorphenoxy)-3'-methylaminomethyl-biphenyl-3-yl]-acetamide;
[2-(3,4-Dichlorophenoxy)-5-thiophen-2-ylbenzyl]-methylamine;
[4-(3,4-Dichlorophenoxy)-4'-fluoro-biphenyl-3-ylmethyl]-methylamine;
[2-(3,4-Dichlorophenoxy)-5-[1,2,3]triazol-1-ylbenzyl]-methylamine;
[2-(3,4-Dichlorophenoxy)-5-[1,2,3]triazol-2-ylbenzyl]-methylamine;
[2-(3,4-Dichlorophenoxy)-5-pyridin-2-ylbenzyl]-methylamine;
[2-(3,4-Dichlorophenoxy)-5-pyridin-3-ylbenzyl]-methylamine;
1-[4-(3,4-Dichlorophenoxy)-3-methylaminomethylphenyl]-1H-pyrazol-3-ylamine;
[2-(3,4-Dichlorophenoxy)-5-pyridin-4-ylbenzyl]-methylamine;
[3-(3,4-Dichlorophenoxy)-biphenyl-4-ylmethyl]-methylamine;
[4-(3,4-Dichlorophenoxy)-4'-methyl-biphenyl-3-ylmethyl]-methylamine;
[2-(3,4-Dichlorophenoxy)-4-thiophen-2-ylbenzyl]-methylamine;
[2-(3,4-dichlorophenoxy)-5-thiazol-2-ylbenzyl]-methylamine;
[2-(3,4-dichlorophenoxy)-5-(1 H-tetrazol-5-yl)benzyl]-methylamine;
[2-(3,4-dichlorophenoxy)-5-furan-3-ylbenzyl]-methylamine;
{1 -[2-(3,4-dichlorophenoxy)-5-[1,2,3]triazol-1 -ylphenyl]ethyl}-methylamine;
{1-[2-(3,4-dichlorophenoxy)-5-[1,2,3]triazol-2-ylphenyl]ethyl}-methylamine;
{1-(2-(3,4-dichlorophenoxy)-5-thiazol-2-ylphenyl]ethyl}-methylamine;
{1 -[2-(3,4-dichlorophenoxy)-4-[1,2,4]triazol-1 -ylphenyl]ethyl}-methylamine;
[2-(3,4-dichlorophenoxy)-5-(5-methylthiophen-2-yl)benzyl]-methylamine;
[2-(3,4-dichlorophenoxy)-5-[1,2,4]triazol-4-ylbenzyl]-methylamine;
1-[4-(3,4-dichlorophenoxy)-3-(methylaminomethyl)phenyl]-pyrrolidin-2-one;
1-[4-(3,4-dichlorophenoxy)-3-(1-methylaminoethyl)phenyl]-pyrrolidin-2-one;
1-[4-(3,4-dichlorophenoxy)-3-(methylaminomethyl)phenyl]-piperidin-2-one;
[2-(3,4-Dichlorophenoxy)-5-fluorobenzyl]-dimethylamine;
[2-(3,4-Dichlorophenoxy)-5-fluorobenzyl]-methylamine;
[2-(3,4-Dichlorophenoxy)-5-trifluoromethylbenzyl]-dimethylamine;
N-[4-(3,4-Dichlorophenoxy)-3-dimethylaminomethylphenyl]-acetamide;
{1-[2-(3,4-Dichlorophenoxy)phenyl]-ethyl}-dimethylamine;
[2-(3,4-Dichlorophenoxy)-4-trifluoromethylbenzyl]-dimethylamine;
[2-(3,4-Dichlorophenoxy)-4-trifluoromethylbenzyl]-methylamine;
[4-Chloro-2-(3,4-dichlorophenoxy)-benzyl]-methylamine;
{1-[2-(3,4-Dichlorophenoxy)-5-fluorophenyl]-ethyl}-methylamine;
{1-[2-(3,4-Dichlorophenoxy)phenyl}-ethyl}-methylamine;
{1-[2-(4-Chlorophenoxy)phenyl]ethyl}-methylamine;
[2-(3,4-Dichlorophenoxy)-5-methoxybenzyl]-methylamine;
[2-(4-Chlorophenoxy)-5-fluorobenzyl]-methylamine;
{1-[2-(4-Chlorophenoxy)-5-fluorophenyl]-ethyl}-methylamine;
[2-(3,4-Dichlorophenoxy)-5-methylbenzyl]-dimethylamine;
[4-Bromo-2-(3,4-dichlorophenoxy)-benzyl]-methylamine;
[5-Bromo-2-(3,4-dichlorophenoxy)-benzyl]-methylamine;
[2-(3,4-Dichlorophenoxy)-4,5-dimethoxybenzyl]-methylamine;
[2-(3,4-Dichlorophenoxy)-4-methoxybenzyl]-dimethylamine;
4-(3,4-Dichlorophenoxy)-3-methylaminomethyl-benzonitrile;
[2-(3,4-Dichlorophenoxy)-4,5-dimethylbenzyl]-methylamine;
3-(3,4-Dichlorphenoxy)-4-methylaminomethyl-benzonitrile;
(+)-{1-[2-(3,4-Dichlorophenoxy)-5-fluorophenyl]-ethyl}-methylamine;
(-)-{1-[2-(3,4-Dichlorophenoxy)-5-fluorophenyl]-ethyl}-methylamine;
[2-(3,4-Dichlorophenoxy)-5-trifluoromethyl-benzyl]-methylamine;
[2-(3,4-Dichlorophenoxy)-4-methoxybenzyl]-methylamine;
[2-(4-Chloro-3-fluorophenoxy)-5-fluorobenzyl]-methylamine;
[2-(3-Chloro-4-fluorophenoxy)-5-fluorobenzyl]-methylamine;
(+/-)-2-[2-(3,4-Dichlorophenoxy)-5-fluorophenyl]-pyrrolidine;
(-)-2-[2-(3,4-Dichlorophenoxy)-5-fluorophenyl]-pyrrolidine;
(+)-2-[2-(3,4-Dichlorophenoxy)-5-fluorophenyl]-pyrrolidine; and
2-[2-(3,4-Dichlorophenoxy)-5-fluorophenyl]-N-methylpyrrolidine.

10. A use according to claim 5, wherein the compound of formula I or formula XXX, respectively, or pharmaceutically acceptable salt thereof, is selected from the following compounds and their pharmaceutically acceptable salts:
[4-(3,4-Dichlorophenoxy)-biphenyl-3-ylmethyl]-methylamine;
[2-(3,4-Dichlorophenoxy)-5-thiophen-3-ylbenzyl]-methylamine;
[2-(3,4-Dichlorophenoxy)-4-thiophen-3-ylbenzyl]-methylamine;
[2-(3,4-Dichlorophenoxy)-4-furan-2-ylbenzyl]-methylamine;
[2-(3,4-Dichlorophenoxy)-5-furan-2-ylbenzyl]-methylamine;
N-[4'-(3,4-Dichlorphenoxy)-3'-methylaminomethyl-biphenyl-3-yl]-acetamide;
[2-(3,4-Dichlorophenoxy)-5-thiophen-2-ylbenzyl]-methylamine;
[4-(3,4-Dichlorophenoxy)-4'-fluoro-biphenyl-3-ylmethyl]-methylamine;
[2-(3,4-Dichlorophenoxy)-5-[1,2,3]triazol-1-ylbenzyl]-methylamine;
[2-(3,4-Dichlorophenoxy)-5-[1,2,3]triazol-2-ylbenzyl]-methylamine;
[2-(3,4-Dichlorophenoxy)-5-pyridin-2-ylbenzyl]-methylamine;
[2-(3,4-Dichlorophenoxy)-5-pyridin-3-ylbenzyl]-methylamine;
1-[4-(3,4-Dichlorophenoxy)-3-methylaminomethylphenyl]-1H-pyrazol-3-ylamine;
[2-(3,4-Dichlorophenoxy)-5-pyridin-4-ylbenzyl]-methylamine;
[3-(3,4-Dichlorophenoxy)-biphenyl-4-ylmethyl]-methylamine;
[4-(3,4-Dichlorophenoxy)-4'-methyl-biphenyl-3-ylmethyl]-methylamine;
[2-(3,4-Dichlorophenoxy)-4-thiophen-2-ylbenzyl]-methylamine;
[2-(3,4-dichlorophenoxy)-5-thiazol-2-ylbenzyl]-methylamine;
[2-(3,4-dichlorophenoxy)-5-(1H-tetrazol-5-yl)benzyl]-methylamine;
[2-(3,4-dichlorophenoxy)-5-furan-3-ylbenzyl]-methylamine;
{1 -[2-(3,4-dichlorophenoxy)-5-[1,2,3]triazol-1-ylphenyl]ethyl}-methylamine;
{1-[2-(3,4-dichlorophenoxy)-5-[1,2,3]triazol-2-ylphenyl]ethyl}-methylamine;
{1-[2-(3,4-dichlorophenoxy)-5-thiazol-2-ylphenyl]ethyl}-methylamine;
{1 -[2-(3,4-dichlorophenoxy)-4-[1,2,4]triazol-1-ylphenyl]ethyl}-methylamine;
[2-(3,4-dichlorophenoxy)-5-(5-methylthiophen-2-yl)benzyl]-methylamine;
[2-(3,4-dichlorophenoxy)-5-[1,2,4]triazol-4-ylbenzyl]-methylamine;
1-[4-(3,4-dichlorophenoxy)-3-(methylaminomethyl)phenyl]-pyrrolidin-2-one;
1-[4-(3,4-dichlorophenoxy)-3-(1-methylaminoethyl)phenyl]-pyrrolidin-2-one;
1-[4-(3,4-dichlorophenoxy)-3-(methylaminomethyl)phenyl]-piperidin-2-one;
[2-(3,4-Dichlorophenoxy)-5-fluorobenzyl]-dimethylamine;
[2-(3,4-Dichlorophenoxy)-5-fluorobenzyl]-methylamine;
[2-(3,4-Dichlorophenoxy)-5-trifluoromethylbenzyl]-dimethylamine;
N-[4-(3,4-Dichlorophenoxy)-3-dimethylaminomethylphenyl]-acetamide;
{1-[2-(3,4-Dichlorophenoxy)phenyl]-ethyl}-dimethylamine;
[2-(3,4-Dichlorophenoxy)-4-trifluoromethylbenzyl]-dimethylamine;
[2-(3,4-Dichlorophenoxy)-4-trifluoromethylbenzyl]-methylamine;
[4-Chloro-2-(3,4-dichlorophenoxy)-benzyl]-methylamine;
{1-[2-(3,4-Dichlorophenoxy)-5-fluorophenyl]-ethyl}-methylam ine;
{1-[2-(3,4-Dichlorophenoxy)phenyl}-ethyl}-methylamine;
{1-[2-(4-Chlorophenoxy)phenyl]ethyl}-methylamine;
[2-(3,4-Dichlorophenoxy)-5-methoxybenzyl]-methylamine;
[2-(4-Chlorophenoxy)-5-fluorobenzyl]-methylamine;
{1-[2-(4-Chlorophenoxy)-5-fluorophenyl]-ethyl}-methylamine;
[2-(3,4-Dichlorophenoxy)-5-methylbenzyl]-dimethylamine;
[4-Bromo-2-(3,4-dichlorophenoxy)-benzyl]-methylamine;
[5-Bromo-2-(3,4-dichlorophenoxy)-benzyl]-methylamine;
[2-(3,4-Dichlorophenoxy)-4,5-dimethoxybenzyl]-methylamine;
[2-(3,4-Dichlorophenoxy)-4-methoxybenzyl]-dimethylamine;
4-(3,4-Dichlorophenoxy)-3-methylaminomethyl-benzonitrile;
[2-(3,4-Dichlorophenoxy)-4,5-dimethylbenzyl]-methylamine;
3-(3,4-Dichlorphenoxy)-4-methylaminomethyl-benzonitrile;
(+)-{1-[2-(3,4-Dichlorophenoxy)-5-fluorophenyl]-ethyl}-methylamine;
(-)-{1-[2-(3,4-Dichlorophenoxy)-5-fluorophenyl]-ethyl}-methylamine;
[2-(3,4-Dichlorophenoxy)-5-trifluoromethyl-benzyl]-methylamine;
[2-(3,4-Dichlorophenoxy)-4-methoxybenzyl]-methylamine;
[2-(4-Chloro-3-fluorophenoxy)-5-fluorobenzyl]-methylamine;
[2-(3-Chloro-4-fluorophenoxy)-5-fluorobenzyl]-methylamine;
(+/-)-2-[2-(3,4-Dichlorophenoxy)-5-fluorophenyl]-pyrrolidine;
(-)-2-[2-(3,4-Dichlorophenoxy)-5-fluorophenyl]-pyrrolidine;
(+)-2-[2-(3,4-Dichlorophenoxy)-5-fluorophenyl]-pyrrolidine; and
2-[2-(3,4-Dichlorophenoxy)-5-fluorophenyl]-N-methylpyrrolidine.

11. A product containing (a) an opioid antagonist, or a pharmaceutically acceptable salt thereof; and (b) a compound of the formula I, as depicted and defined below, 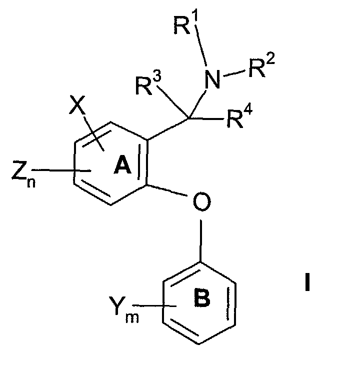 or pharmaceutically acceptable salt thereof, as a combined preparation for simultaneous, separate or sequential use in the treatment of alcoholism or alcohol dependence in a mammal, wherein phenyl ring A and phenyl ring B can each, independently, be replaced by a naphthyl group, and wherein when phenyl ring A is replaced by a naphthyl group, the ethereal oxygen of structure I and the carbon to which R³, R⁴ and NR¹R² are attached, are attached to adjacent ring carbon atoms of the naphthyl group and neither of said adjacent ring carbon atoms is also adjacent to a fused ring carbon atom of said naphthyl group;
n and m are selected, independently, from one, two and three;
R¹ and R² are selected, independently, from hydrogen, (C₁-C₄)alkyl, (C₂-C₄)alkenyl, and (C₂-C₄)alkynyl, or R¹ and R², together with the nitrogen to which they are attached, form a four to eight membered saturated ring containing one or two heteroatoms, including the nitrogen to which R¹ and R² are attached, wherein the second heteroatom, when present, is selected from oxygen, nitrogen and sulfur, and wherein said ring may optionally be substituted at available binding sites with from one to three substituents selected, independently, from hydroxy and (C₁-C₆)alkyl;
R³ and R⁴ are selected, independently, from hydrogen and (C₁-C₄) alkyl optionally substituted with from one to three fluorine atoms, or R³ and R⁴ together with the carbon to which they are attached form a four to eight membered saturated carbocyclic ring, and wherein said ring may optionally be substituted at available binding sites with from one to three substituents selected, independently, from hydroxy and (C₁-C₆)alkyl;
or R² and R³, together with the nitrogen to which R² is attached and the carbon to which R³ is attached, form a four to eight membered saturated ring containing one or two heteroatoms, including the nitrogen to which R² is attached, wherein the second heteroatom, when present, is selected from oxygen, nitrogen and sulfur, and wherein said ring may optionally be substituted at available binding sites with from one to three substituents selected, independently, from hydroxy and (C₁-C₆)alkyl;
each X is selected, independently, from phenyl, heteroaryl and heterocyclic groups, and may be further substituted by hydrogen, halo, (C₁-C₄)alkyl optionally substituted with from one to three fluorine atoms, (C₁-C₄)alkoxy optionally substituted with from one to three fluorine atoms, cyano, nitro, amino, hydroxy, carbonyl, (C₁-C₄)alkylamino, di-[(C₁-C₄)alkyl]amino, NR⁵(C=O)(C₁-C₄)alkyl, SO₂NR⁵R⁶ or SOₚ(C₁-C₆)alkyl, wherein R⁵ and R⁶ are selected, independently, from hydrogen and (C₁-C₆)alkyl, and p is zero, one or two;
each Y is selected, independently, from hydrogen, halo, (C₁-C₄)alkyl optionally substituted with from one to three fluorine atoms, (C₁-C₄)alkoxy optionally substituted with from one to three fluorine atoms, cyano, nitro, amino, (C₁-C₄)alkylamino, di-[(C₁-C₄)alkyl]amino, NR⁵(C=O)(C₁-C₄)alkyl, SO₂NR⁵R⁶ and SOₚ(C₁-C₆)alkyl, wherein R⁵ and R⁶ are selected, independently, from hydrogen and (C₁-C₆)alkyl, and p is zero, one or two; and
each Z is selected independently from hydrogen, halo, (C₁-C₄)alkyl optionally substituted with from one to three fluorine atoms, and (C₁-C₄)alkoxy;
or a compound of the formula XXX, as depicted and defined below, 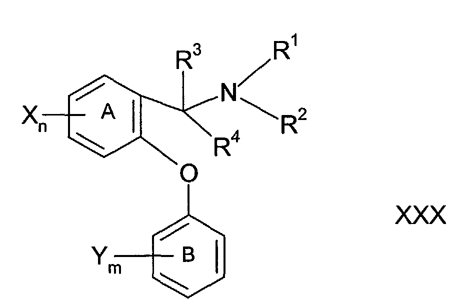 or pharmaceutically acceptable salt thereof, wherein phenyl ring A and phenyl ring B can each, independently, be replaced by a naphthyl group, and wherein when phenyl ring A is replaced by a naphthyl group, the ethereal oxygen of structure I and the carbon to which R³, R⁴ and NR¹R² are attached, are attached to adjacent ring carbon atoms of the naphthyl group and neither of said adjacent ring carbon atoms is also adjacent to a fused ring carbon atom of said naphthyl group;
n and m are, selected, independently, from one, two and three;
R¹ and R² are selected, independently, from hydrogen, (C₁-C₄)alkyl, (C₂-C₄)alkenyl, and (C₂-C₄)alkynyl, or R¹ and R², together with the nitrogen to which they are attached, form a four to eight membered saturated ring containing one or two heteroatoms, including the nitrogen to which R¹ and R² are attached, wherein the second heteroatom, when present, is selected from oxygen, nitrogen and sulfur, with the proviso that said ring can not contain two adjacent oxygen atoms or two adjacent sulfur atoms, and wherein said ring may optionally be substituted at available binding sites with from one to three substituents selected, independently, from hydroxy and (C₁-C₆)alkyl;
R³ and R⁴ are selected, independently, from hydrogen and (C₁-C₄) alkyl optionally substituted with from one to three fluorine atoms, or R³ and R⁴, together with the carbon to which they are attached, form a four to eight membered saturated carbocyclic ring, and wherein said ring may optionally be substituted at available binding sites with from one to three substituents selected, independently, from hydroxy and (C₁-C₆)alkyl;
or R² and R³, together with the nitrogen to which R² is attached and the carbon to which R³ is attached, form a four to eight membered saturated ring containing one or two heteroatoms, including the nitrogen to which R² is attached, wherein the second heteroatom, when present, is selected from oxygen, nitrogen and sulfur, with the proviso that said ring can not contain two adjacent oxygen atoms or two adjacent sulfur atoms, and wherein said ring may optionally be substituted at available binding sites with from one to three substituents selected, independently, from hydroxy and (C₁-C₆)alkyl;
each X is selected, independently, from hydrogen, halo, (C₁-C₄)alkyl optionally substituted with from one to three fluorine atoms, (C₁-C₄)alkoxy optionally substituted with from one to three fluorine atoms, cyano, nitro, amino, (C₁-C₄)alkylamino, di-[(C₁-C₄)alkyl]amino, NR⁵(C=O)(C₁-C₄)alkyl, SO₂NR⁵R⁶ and SOₚ(C₁-C₆)alkyl, wherein R⁵ and R⁶ are selected, independently, from hydrogen and (C₁-C₆)alkyl, and p is zero, one or two; and
each Y is selected, independently, from hydrogen, (C₁-C₆)alkyl and halo;
with the proviso that: (1) no more than one of NR¹R², CR³R⁴ and R²NCR³ can form a ring; and (2) at least one X must be other than hydrogen when (i) R³ and R⁴ are both hydrogen, (ii) R¹ and R² are selected, independently, from hydrogen and (C₁-C₄)alkyl, and (iii) ring B is mono- or disubstituted with, respectively, one or two halo groups;
or a compound of the formula XXXI or XXXII, respectively, as depicted below, 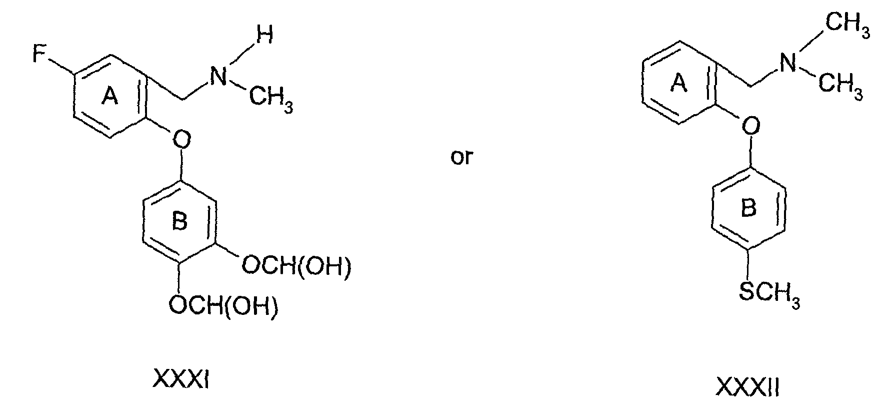 or pharmaceutically acceptable salt thereof; wherein the active agents "a" and "b" above are present in amounts that render the combination of the two agents effective in treating alcoholism or alcohol dependence.
